(19) Europäisches Patentamt
European Patent Office
Office européen des brevets

(11) Publication number : 0 439 330 A2

(12) EUROPEAN PATENT APPLICATION

(21) Application number : 91300479.2

(22) Date of filing : 22.01.91

(51) Int. Cl.[5] : C12Q 1/68, // C07H21/00

(30) Priority : 25.01.90 GB 9001764
21.11.90 GB 9025283

(43) Date of publication of application :
31.07.91 Bulletin 91/31

(84) Designated Contracting States :
AT BE CH DE DK ES FR GB GR IT LI LU NL SE

(71) Applicant : IMPERIAL CHEMICAL INDUSTRIES PLC
Imperial Chemical House, Millbank
London SW1P 3JF (GB)

(72) Inventor : Copley, Clive Graham
43 Sycamore Crescent
Macclesfield, Cheshire (GB)
Inventor : Anwar, Rashida
34 Shelley Avenue, Wincham
Northwich, Cheshire (GB)
Inventor : Mcpheat, William Leishman
4 Bollin drive
Congleton, Cheshire (GB)
Inventor : Markham, Alexander Fred
25 Booth Bed Lane, Goostrey
Crewe, Cheshire (GB)
Inventor : Smith, John Craig
14 Ascol Drive, Plumley
Nr Knutsford, Cheshire (GB)

(74) Representative : Mack, John Richard et al
Imperial Chemical Industries PLC Legal
Department: Patents PO Box 6 Bessemer
Road
Welwyn Garden City Hertfordshire AL7 1HD
(GB)

# (54) Amplification methods.

(57) A method for the amplification of at least a part of a target nucleic acid fragment/vectorette unit, said part comprising i) unknown sequence, ii) a first priming region of known nucleotide sequence for hybridisation with a first primer and iii) a vectorette portion at least one strand of which has a nucleic acid sequence which in either its double stranded complementary form or its single stranded form is capable of being bound by protein, which method comprises treating the target nucleic acid fragment/vectorette unit, together or sequentially, with appropriate nucleoside triphosphates and an agent for polymerisation of the nucleoside triphosphates under hybridising conditions, such that an extension product of a first priming region is synthesised complementary to a single stranded target nucleic acid/vectorette unit having a first priming region to which is hybridised a first primer selected so as to be substantially complementary to the first priming region, whereas no such extension product is synthesised complementary to single stranded target nucleic acid fragment/vectorette units having no such first priming region.

EP 0 439 330 A2

# AMPLIFICATION METHODS

The present invention relates to methods for the amplification of nucleotide sequences and kits therefor. Such methods are of interest for example in relation to the amplification of sequences only a portion of which is known and enables long nucleotide sequences to be rapidly and efficiently sequenced.

In our European Patent Application No. 89307672.9, publication no. 0356021 we describe and claim a method for the amplification of a nucleic acid fragment, comprising unknown sequence, by primer extension which method comprises cleaving a target nucleic acid to obtain target nucleic acid fragments, one of said fragments containing an initiating priming region of known nucleotide sequence for hybridisation with an initiating primer, preparing target nucleic acid fragment/vectorette units from the target nucleic acid fragments by ligation each unit having a vectorette priming region of known sequence for hybridisation with a vectorette primer, and treating the target nucleic acid fragment/vectorette units, together or sequentially, with appropriate nucleoside triphosphates and an agent for polymerisation of the nucleoside triphosphates under hybridising conditions, such that an extension product of an initiating primer is synthesised complementary to a single stranded target nucleic acid/vectorette unit having an initiating priming region to which is hybridised an initiating primer selected so as to be substantially complementary to the initiating priming region, whereas no such extension product is synthesised complementary to single stranded target nucleic acid fragment/vectorette units having no such initiating priming region.

This method including its preferred features detailed below is referred to herein as the Chemical Genetics technique.

If desired the extension product referred to above may be subjected to amplification in the presence of a vectorette primer which is selected so as to be substantially complementary to the vectorette priming region.

The target nucleic acid fragment/vectorette units are thus treated with initiating primer and, if the initiating primer extension product is to be amplified for example as described by R.K. Saiki et al, Science 239, 487-491 (1987), additionally treated with vectorette primer. Where no vectorette primer is used, arithmetical or linear amplification (hereinafter referred to as linear amplification) may be achieved by hybridisation of the initiating primer to the initiating priming region followed by primer extension in the presence of appropriate nucleoside triphosphates and an agent for polymerisation of the nucleoside triphosphates, under hybridising conditions and denaturation. This process of priming, primer extension and denaturation may be repeated as many times as appropriate to achieve the desired level of amplification. Preferably, however, amplification is effected in the presence of both initiating and vectorette primers by the use of the PCR technique referred to hereinafter.

In a preferred embodiment of our aforementioned European Patent Application the invention is effected such that the synthesis of primer extension products or preferably vectorette primer amplification products is dependent upon the initial synthesis of an extension product of the initiating primer.

This may, for example, be achieved by linear amplification or preferably by the use of a vectorette primer which is only capable of hybridising to the extension product of the initiating primer. Thus advantageously the vectorette portion of the target nucleic acid fragment/vectorette unit comprises a double stranded portion having first and second strands, the first strand having a terminal polymerisation blocking moiety and the second strand, which is ligated to that strand of the target nucleic acid fragment containing the initiating priming region, carrying a single stranded portion, the terminal polymerisation blocking moiety being effective to prevent extension of the first strand to form a complement to the said single stranded portion of the second strand in the presence of appropriate nucleoside triphosphates and an agent for the polymerisation of the nucleoside triphosphates under hybridising conditions. The vectorette primer is thus incapable of hybridising to the target nucleic acid fragment/vectorette unit, but is capable of hybridising to the extension product of the initiating primer. Thus no vectorette primer extension products are obtained until generation of an extension product of the initiating primer, following which the extension products obtained normally include a portion complementary to the known nucleotide sequence(s) of the single stranded portion of the second strand. This advantageous embodiment is illustrated hereinafter in Figure 1.

The polymerisation blocking moiety is effective to prevent polymerisation of a nucleotide sequence from a primer to form the complement of a template nucleotide sequence in the presence of an agent for polymerisation of nucleoside triphosphates such as a DNA polymerase, for example Klenow fragment of E. coli DNA polymerase I, T7 DNA polymerase or Taq DNA polymerase. The polymerisation blocking moiety may be any convenient group known for this purpose such as an appropriate modified nucleoside for example a dideoxynucleoside or a 3'- deoxynucleoside such as cordycepin or a 3'-amino or a 3'-thio functionality.

In a further especially preferred embodiment the vectorette portion of the target nucleic acid fragment/vectorette unit comprises a double stranded portion having first and second strands, the second strand of the vectorette portion being ligated to that strand of the target nucleic acid fragment which contains the initiating priming

region, and the nucleotide sequence of the first strand, second strand and vectorette primer being selected such that the vectorette primer is capable of hybridising to the complement of the second strand but not to the first strand under the same hybridisation conditions. It will be appreciated that the presence of a polymerisation blocking moiety in this embodiment is not necessary. It will further be appreciated, in this case, that the sequence of the vectorette primer will be substantially the same as the sequence of at least a portion of the second strand of the vectorette. This especially preferred embodiment of the invention is discussed further hereinafter in relation to Figure 2.

The Chemical Genetics technique is of wide applicability. Thus for example the Chemical Genetics technique may be employed to identify nucleotide sequences charateristic of a particular microorganism such as a microorganism responsible for disease in plants or animals, particularly humans, such microorganism being for example a fungus, yeast, bacterium or virus as well as a parasite (such as plasmodium (malaria) or trypanosome (sleeping sickness). The method of the Chemical Genetics technique may also be employed to identify nucleotide sequences responsible for drug, for example antibiotic, resistance in a given organism. Thus the technique enables the production of probes diagnostic of disease in animals or plants and additionally enables the production of probes diagnostic of drug, for example antibiotic, resistance.

The Chemical Genetics technique may also be employed for example 1) to detect nucleotide sequence variations, for example point mutations, responsible for genetic disorders in plants, but especially in animals, particularly humans ; 2) to detect nucleotide sequence variations, for example deletions, responsible for neoplastic disease in animals, particularly humans ; 3) to detect nucleotide sequence variations responsible for predispositions to disease or disorders in plants but especially in animals, particularly humans ; and 4) to detect nucleotide sequence variations responsible for a specific characteristic such as a desirable characteristic, for example in plants such as flower colour, crop yield or herbicide resistance.

The present invention is based at least in part on the discovery that the vectorette portion of the target nucleic acid fragment/vectorette portion of the target nucleic acid fragment/vectorette unit may include a nucleic acid sequence which in either its double stranded complementary form or its single stranded form is capable of being bound by protein.

Thus according to one feature of the present invention there is provided a method for the amplification of at least a part of a target nucleic acid fragment/vectorette unit, said part comprising i) unknown sequence, ii) a first priming region of known nucleotide sequence for hybridisation with a first primer and iii) a vectorette portion at least one strand of which has a nucleic acid sequence which in either its double stranded complementary form or its single stranded form is capable of being bound by protein, which method comprises treating the target nucleic acid fragment/vectorette unit, together or sequentially, with appropriate nucleoside triphosphates and an agent for polymerisation of the nucleoside triphosphates under hybridising conditions, such that an extension product of a first priming region is synthesised complementary to a single stranded target nucleic acid/vectorette unit having a first priming region to which is hybridised a first primer selected so as to be substantially complementary to the first priming region, whereas no such extension product is synthesised complementary to single stranded target nucleic acid fragment/vectorette units having no such first priming region.

The target nucleic acid fragment/vectorette unit may for example be obtained by cleaving a target nucleic acid to obtain target nucleic acid fragments, one of said fragments containing a first priming region and preparing the aforesaid vectorette unit by ligation of the target nucleic acid fragment and vectorette, the vectorette having at least a portion, at least one strand of which has a nucleic acid sequence which in either its double stranded complementary form or its single stranded form is capable of being bound by protein. If desired the vectorette may also have a priming region of known sequence for hybridisation with a second primer.

It will be understood that the nucleic acid sequence capable of being bound by protein is defined in that either in its double stranded complementary form it is capable of being bound by protein or in its single stranded form it is capable of being bound by protein, but protein will not be capable of binding the said nucleic acid sequence in both such forms. Preferably the nucleic acid sequence is such that it is only capable of being bound by protein when the nucleic acid sequence is in its double stranded complementary form.

As stated above the vectorette portion of the target nucleic acid fragment/vectorette unit comprises a nucleic acid sequence which either in its double stranded complementary form or in single stranded form is capable of being bound by protein. Such sequences in double stranded form may for example include cAMP-binding protein sites capable of stimulating secondary responses, recombination protein binding sites, restriction enzyme binding sites and binding sites for regulatory proteins which are then capable of being activated by specific environmental signals, as well as single stranded specific binding protein sites capable of differentiating single and double stranded termini, but particularly promoters capable of directing in vitro transcription/translation of adjoining sequences. A particular example of the nucleic acid protein binding sequence is a nucleic acid capable of binding an RNA polymerase, for example a nucleic acid sequence capable of binding SP6 or T7 RNA polymerase. The use of such a polymerase enables the production of RNA transcripts which may for

example be used as nucleotide sequencing templates, used as hybridisation probes or in transcription mapping.

It will be appreciated that the Chemical Genetics technique as hereinbefore defined enables the generation of an ordered series of overlapping nucleic acid fragments from any desired source of nucleic acid sequence. The technique may be performed by determining the sequence of amplified product obtained in order to prepare a further primer for use in the next "step" of the technique. In this way the operator of the technique may proceed in a stepwise fashion along a nucleic acid sequence by repeating the steps of forming an amplified product, sequencing and preparing a new primer for the next step. The technique is however capable of generating large nucleic acid fragments (for example over 2kb) and in such cases it may be preferred merely to determine sufficient terminal sequence to prepare primer for the next step. In this regard the present invention is also based on the discovery that the above problem may be mitigated by cleaving the target nucleic acid fragment/vectorette unit in a region of unknown sequence in the target nucleic acid fragment portion and ligating a vectorette to the cleaved target nucleic acid portion to form a vectorette unit having a vectorette portion at each terminus.

Thus according to a further feature of the present invention there is provided a method for the production of a target nucleic acid fragment/vectorette unit having a vectorette portion at each terminus of the said unit which method comprises cleaving a target nucleic acid fragment/vectorette unit in a region of unknown sequence in the target nucleic acid fragment portion of the said vectorette unit and ligating a vectorette to the cleaved target nucleic acid portion obtained to form a vectorette unit having a vectorette portion at each terminus of the said unit.

The vectorette for ligation to the cleaved target nucleic acid portion preferably comprises a nucleic acid sequence which in either its double stranded complementary form or its single stranded form is capable of being bound by protein. Examples of such nucleic acid sequences are as hereinbefore described.

Thus the target nucleic acid fragment/vectorette unit having a vectorette portion at each terminus of the unit may be subjected to amplification in any desired manner as referred to hereinafter, for example by polymerase chain reaction (PCR) techniques as for example described by R.K. Saiki et al in Science 239, 487-491 (1987) and in US Patent Specification Nos 4,683,195 and 4,683,202 or by the amplification processes described in PCT Patent Publication WO 87/06270 (or Biotechnology Vol 6, October 1988), PCT Patent Publication WO 88/10315 or PCT Patent Publication WO 89/01050.

It will be appreciated that amplification by PCR is not necessary. Thus for example a double stranded promoter sequence, for example a double stranded RNA polymerase promoter sequence may be formed by primer extension of the first primer in the absence of a vectorette primer, subsequent amplification of the target sequence being effected by repeated generation of the transcribed sequence, for example RNA transcript.

Thus in a further embodiment of the present invention amplification of at least a part of the vectorette unit is effected by a) primer extension of a first primer, said first primer being hybridised to the first priming region of a nucleic acid sequence which sequence comprises i) unknown sequence and ii) a promoter sequence ; followed by b) repeated generation of nucleic acid sequence under the control of the said promoter sequence.

The vectorette portion of the target nucleic acid fragment/vectorette unit conveniently comprises at least one nucleic acid sequence which is capable of being bound by a restriction enzyme. The nucleic acid sequence is conveniently double stranded, the complementary strand preferably being formed by extension of the first primer. Any convenient number of such nucleic acid sequences, such as 1, 2 , 3 , 4, 5, or 6, in particular 2, 3 or 4, for example 3, may be comprised in the vectorette portion. The nucleic acid sequence may be the recognition sequence for any chosen restriction enzyme. Examples of individual restriction endonucleases include those detailed in Nucleic Acids Research, Sequences Supplement, Volume 16, 1988 page r271-r313 and in "Current Protocols in Molecular Biology 1987-1988, Edited by Ausubel F.M., Brent R., Kingston R.E., Moor D.D, Smith J.A., Seidman J.G. and Struhl K., Wiley Interscience, Section 3, Table 3.1-1. The presence of at least one restriction site in the vectorette portion provides, after cleavage, an end of known sequence which may be particularly useful for insertion of the nucleic acid fragment/vectorette unit into a vector suitable for cloning/sub cloning, either for sequencing or for expression. Any convenient vector may be used, for example as selected from the catalogues of Clontech Laboratories, 4030 Fabian Way, Palo Alto, California 94303, USA or Promega, 2800 Woods Hollow Road, Madison, Wisconsin 53711-5399, USA. Convenient vectors include plasmid and bacteriophage vectors, for example M13. Where after cleavage the nucleic acid fragment/vectorette unit has dissimilar ends this is particularly convenient for insertion into a vector since its orientation can be readily determined. Alternatively after cleavage the nucleic acid fragment/vectorette may be spliced or linked to other nucleic acid sequence(s), for example comprising any desired function, such as hereinbefore described. It will be appreciated that any convenient combination of nucleic acid sequences for binding to proteins of similar or dissimilar function may be comprised in the vectorette portion of the nucleic acid fragment/vectorette unit. Thus for example the vectorette unit may comprise both site(s) for recognition by restriction enzymes and site(s) for recognition by other protein(s) such as promoter(s).

It is preferred that the vectorette unit is such that the synthesis of amplification products comprising nucleic

acid sequence which either in its double stranded complementary form or in its single stranded form is capable of being bound by protein, is dependent upon the initial synthesis of an extension product of the first primer. Thus for example where it is desired to use a target nucleic acid fragment/vectorette unit having two vectorette portions (one vectorette portion at each terminus of the vectorette unit) at least one of the vectorette portions is such that the synthesis of amplification products is dependent upon the initial synthesis of an extension product of a primer capable of hybridising to the other of the said vectorette portions. Thus advantageously at least one vectorette portion of the target nucleic acid fragment/vectorette unit comprises a double stranded portion having first and second strands, the first strand having a terminal polymerisation blocking moiety and the second strand carrying a single stranded portion which comprises nucleic acid sequence either in its double stranded complementary form or in its single stranded form is capable of being bound by protein, the second strand being ligated to that strand of the target nucleic acid fragment containing a first priming region for hybridisation with a first primer, the terminal polymerisation blocking moiety being effective to prevent extension of the first strand to form a complement to the said single stranded portion of the second strand in the presence of appropriate nucleoside triphosphates and an agent for the polymerisation of the nucleoside triphosphates under hybridising conditions. A second primer may thus be designed which is incapable of hybridising to the target nucleic acid fragment/vectorette unit, but is capable of hybridising to the extension product of the first primer. Thus no second primer extension products are obtained until generation of an extension product of the first primer, following which the extension products obtained normally include a portion complementary to the known nucleotide sequence(s) of the single stranded portion of the second strand. This advantageous embodiment is illustrated hereinafter in Figure 1.

The polymerisation blocking moiety is effective to prevent polymerisation of a nucleotide sequence from a primer to form the complement of a template nucleotide sequence in the presence of an agent for polymerisation of nucleoside triphosphates such as a DNA polymerase, for example Klenow fragment of E. coli DNA polymerase I, T7 DNA polymerase or Taq DNA polymerase. The polymerisation blocking moiety may be any convenient group known for this purpose such as an appropriate modified nucleoside for example a dideoxynucleoside or a 3'- deoxynucleoside such as cordycepin or a 3' amino or a 3'-thio functionality. Polymerisation blocking moieties are also exemplified by those detailed in our British Patent Application No. 8920097.6.

In a further especially preferred embodiment of the present invention at least one vectorette portion of the target nucleic acid fragment/vectorette unit comprises a double stranded portion having first and second strands, the second strand of the vectorette portion being ligated to that strand of the target nucleic acid fragment which contains a first priming region, and the nucleotide sequence of the first strand, second strand and second primer being selected such that the second primer is capable of hybridising to the complement of the second strand but not to the first strand under the same hybridisation conditions. It will be appreciated that the presence of a polymerisation blocking moiety in this embodiment is not necessary. It will further be appreciated, in this case, that the sequence of the second primer will be substantially the same as the sequence of at least a portion of the second strand of the vectorette. This especially preferred embodiment of the invention is discussed further hereinafter in relation to Figure 2. In this especially preferred embodiment therefore the vectorette in its double stranded complementary form comprises at least a region of non-complementarity. This region of non-complementarity is conveniently positioned between two regions of complementary sequence. In this embodiment of the method of the present invention the nucleic acid sequence which either in its double stranded complementary form or in its single stranded form is capable of being bound by protein will be present in at least one strand of the non-complementary region of the vectorette. If desired each strand of the non-complementary region of the vectorette may comprise a different nucleic acid sequence, each sequence in either its double stranded complementary form or its single stranded form being capable of being bound by protein (normally a different protein).

Where the vectorette portion of the target nucleic acid fragment/vectorette unit comprises at least one nucleic acid sequence which is capable of being bound by a restriction enzyme, one strand of the vectorette may for example comprise the recognition sequence for up to three, conveniently up to three restriction endonuclease(s). The restriction endonucleases are preferably selected to recognise the nucleic acid sequence only when this has been made double stranded by extension of the first primer. The cleaved nucleic acid fragment/vectorette unit may then be sub-cloned as previously described.

A further preferred embodiment of the present invention comprises the preparation of a plurality of different functional vectorette libraries for use with the same single first primer, each vectorette library being prepared by cleaving target nucleic acid at different cleavage sites and preparing target nucleic acid fragment/vectorette units from the target nucleic acid fragments by ligation whereby to form the said functional vectorette library ; treating each functional vectorette library either separately or together with appropriate nucleoside triphosphates and an agent for polymerisation of the nucleoside triphosphates under hybridising conditions whereby to obtain a plurality of first primer extension products based on use of the same single initiating primer. The

size of such extension products will be determined by the distance from the first primer to the closest 3' site for the particular cleavage means, for example restriction enzyme used to construct that particular library.

If desired one or more of said first primer extension products may be isolated and/or sequenced or at least a portion of the extension product may be sequenced as hereinbefore described. Thus for example this embodiment may be conveniently used to identify a desired, normally the longest, target nucleic acid fragment containing a first priming region, so that the 3' terminal end may be sequenced conveniently as hereinbefore described in order to provide a new start point for further use of the method of the present invention. The sequence of the 3' terminal end of the aforementioned longest target nucleic acid fragment may thus become the first priming region of a new target nucleic acid fragment for a further round of vectorette library multiple first primer extension product formation, identification of the longest target nucleic acid fragment and sequencing.

In selecting a new first priming region on the basis of novel sequence data generated using the method of the invention at the 3' terminal end of a target nucleic acid fragment such sequence data may routinely be compared with the publicly available database compilations of known nucleic acid sequence (for example Genbank, EMBL) so as to ensure that a proposed new first priming region does not by chance closely match a known nucleic acid sequence elsewhere in for example the genomic DNA of interest. This is obviously most likely to occur in those cases where the 3'-terminal end of a particular target nucleic acid fragment happens to comprise repetitive elements such as for example Alu sequences. In such cases it is advantageous to perform the method of the invention on a plurality of vectorette libraries with a given first primer so as to guarantee that at least one of the resulting extension products has a non-repetitive/unique 3'-terminal end for the selection of a further first priming region.

Stepwise progression from one previously unknown first priming region to another along a target nucleic acid, for example human genomic DNA, may conveniently be monitored using samples of the said target nucleic acid separately cleaved to completion with the same restriction endonucleases as used in the preparation of target nucleic acid fragment/vectorette units ("vectorette libraries" as hereinbefore defined) and subjected to agarose gel electrophoresis and Southern Blotting. Probing of the filters so obtained with an initial first primer will reveal a pattern of bands consistent with the various restriction enzyme recognition sites surrounding this initial first priming region in the target nucleic acid. Use of the method of the present invention with a plurality of vectorette libraries and this initial first primer will generate a series of extension products each of whose 3'terminal ends are defined by the position relative to the first priming region of the closest recognition site for the restriction enzyme used to generate the vectorette library in question. Thus a map of the restriction sites to the 3' side of an initial first primer is effectively obtained. Having subsequently selected a second novel first priming region of previously unknown sequence, linkage to the initial first priming region is established by reprobing the above Southern Blot filter with the second novel first primer. The pattern of bands obtained will be identical to that obtained with the initial first primer in those cases where no recognition site for the restriction enzyme in question lies between the first and second first priming regions. In those cases where a recognition site for the restriction enzyme in question does occur between the first priming regions as judged by the appearance of smaller extension products in the corresponding vectorette library,then a fragment of different size will normally be observed on reprobing the Southern Blot filter with the second first primer. By repetition of this method consistency, accuracy and reliability of stepwise progression from one initiating priming region to another along a target nucleic acid is maintained and assured.

It will be appreciated that the sequence of the 3'-terminal ends of all the plurality of first primer extension products may be easily obtained using the same vectorette primer or nested vectorette primers for sequencing by methods known per se. In this way the entire sequence of an unknown segment of target DNA nucleic acid may be determined in a facile and systematic manner and with much greater convenience than for example using M13 "Shotgun" cloning. This is because the first primer extension products can be ordered by size and therefore the order of their sequences in the original target nucleic acid becomes apparent. Each first primer extension product shares a 5'-extremity determined by the first primer and a 3'-extremity determined by the closest 3'-site for the particular cleavage means, for example restriction enzyme, used in the synthesis of that particular vectorette library.

Thus in a preferred embodiment of the present invention any or all of the first primer extension product(s) obtained is(are) sequenced at least at the end(s) distal to a given first primer so as to determine the sequence of a further first primer whereby to obtain further first primer extension products based on primer extension of the further first primer.

In a further preferred embodiment according to the present invention a first primer extension product or portion thereof is sequenced whereby to characterise the said extension product or portion thereof.

According to a further feature of the present invention there is provided a kit for the amplification of a nucleic acid fragment of unknown sequence by primer extension, which kit comprises :-
a vectorette adapted for ligation to a target nucleic acid fragment or for ligation to a target nucleic acid fragment/vectorette unit, said fragment or vectorette unit having been obtained by use of a means for cleaving a target nucleic acid whereby to form in use a target nucleic acid fragment/vectorette unit having either a single terminal vectorette or a vectorette at each terminus of the target nucleic acid fragment said vectorette being such that at least one strand thereof has a nucleic acid sequence which in either its double stranded complementary form or its single stranded form is capable of being bound by protein ; and written or printed instructions for use of the kit. The kit will also advantageously additionally contain one or more selected from the following :-

The kit of the present invention will preferably additionally contain the appropriate protein which is capable of binding the nucleic acid sequence present in the vectorette. Thus for example where the nucleic acid sequence is capable of being bound by an RNA polymerase, the kit may additionally contain the RNA polymerase, for example SP6 or T7 RNA polymerase.

1) means for cleaving a target nucleic acid at a specific site to obtain a target nucleic acid fragment or to obtain a target nucleic acid fragment/vectorette unit ;

2) each of four different nucleoside triphosphates ; and

3) an agent for polymerisation of the nucleoside triphosphates in (2).

It will be appreciated that the means for cleaving target nucleic acid at a specific site to obtain a target nucleic acid fragment or to obtain a target nucleic acid fragment/vectorette unit need not necessarily be the same as the means for producing the terminus of the vectorette provided that the vectorette is adapted for ligation to either the target nucleic acid fragment or the target nucleic acid fragment/vectorette unit (having only a single terminal vectorette unit).

The kit may additionally comprise appropriate first and/or vectorette primers, such primers may include primers for hybridisaton as described in relation to the Chemical Genetics technique (as hereinbefore described) and/or primers for hybridisation to the nucleic acid sequence which in either its double stranded complementary form or its single stranded form is capable of being bound by protein.

A vectorette priming region may be present or absent from the vectorette portion of a target nucleic acid fragment/vectorette unit as hereinafter defined. Thus the vectorette (2) in the kit of the present invention may itself contain no vectorette priming region. Thus such units may for example have a vectorette priming region which only arises as a result of primer extension of a first primer as described hereinafter. Further and preferably the vectorette portion of the target nucleic acid fragment/vectorette unit may comprise no vectorette priming region, amplification being effected by for example the use of a nucleic acid sequence present in the vectorette capable of being bound by an agent for polymerisation of nucleoside triphosphates, for example a DNA polymerase or an RNA polymerase (for example SP6 or T7 RNA polymerase). References throughout the specification to "target nucleic acid fragment/vectorette unit" (also termed "vectorette unit") as hereinafter defined are to be so understood.

The kit may, if desired, comprise a vectorette primer having a nucleotide sequence substantially complementary to a vectorette priming region of the target nucleic acid fragment/vectorette unit. The presence of such a vectorette primer in the kit of the present invention will enable amplification of the target nucleic acid fragment/vectorette unit to be effected by PCR techniques (as hereinafter defined) if this is desired.

The kit may also contain a series of "nested" vectorette primers (as hereinafter defined) which may be used, for example, for secondary amplification reactions where necessary or desired and/or for direct sequencing of the products of amplification as has been described in Proc. Natl. Acad. Sci. USA 85, 7652-7656 (1988) by U.B. Gyllenstein and H. Ehrlich. It will be appreciated that all such vectorette primers may well be useful as direct sequencing primers for the distal ends of fragments obtained by linear amplification using a first primer alone.

Since the target nucleic acid to be investigated will normally be peculiar to the user of the kit, first primer(s) will not normally be present in the kit, but may be prepared by the user of the kit.

If desired however the kit of the present invention may additionally contain first primer(s) and also, if desired, nested first primer(s).

The kit of the present invention may also advantageously contain buffers for performing the method of the invention, a particular feature of the kit being for example the presence of buffers for varying the potassium, magnesium and nucleoside triphosphate(s) concentrations of the reaction mixture. These latter buffers may be desirable for determining the optimum conditions for subsequent cycles of the method of the invention.

Advantageously the kit will comprise more than one (a plurality of) means for cleaving a target nucleic acid or target nucleic acid fragment/vectorette each at specific unit sites. Where a plurality of such means are present, the kit will normally comprise a different vectorette for each such means present in order to permit formation of target nucleic acid fragment/vectorette units in respect of each set of target nucleic acid fragments or target nucleic acid fragment/vectorette units (each comprising a single vectorette portion) which may be obtained. Although the invention is not limited to this application, the plurality of different vectorettes will advantageously share sequences complementary to a vectorette primer. In such cases a single vectorette primer will provide amplification from a plurality of target nucleic acid cleavage sites.

Thus in a preferred embodiment the kit of the present invention additionally contains one or more selected from the following :- first primer(s), nested vectorette primer(s), nested first primer(s), sequencing primers, buffers for performing the method of the present invention, and buffers for varying the magnesium, potassium and nucleoside triphosphate concentrations.

According to a further feature of the present invention there is provided a vectorette library kit for the amplification of a nucleic acid fragment of unknown sequence by primer extension, which kit comprises :-

1) at least one vectorette library each library comprising a set of target nucleic acid fragment/vectorette units obtained from nucleotide sequence of an individual member of a species of animal, plant or microorganism, each unit comprising target nucleic acid having either one or two terminal vectorette portions, at least one of said portions having at least one strand of nucleic acid sequence which in either its double stranded complementary form or its single stranded form is capable of being bound by protein ; and

2) a first primer or primers for hybridisation to the first priming region of the target nucleic acid fragment/vectorette units.

The vectorette library kit will preferably comprise a plurality of vectorette libraries.

The vectorette library kit may also additionally include any one or more of the features detailed above in relation to the previously described kit of the invention.

The target nucleic acid fragment/vectorette units may be prepared for example either directly from the desired species or indirectly from such a species after initial cloning in plasmid, phage, cosmid or yeast artificial chromosome (YAC) vectors. The species of animal, plant or microorganism employed is preferably human, but may be any other animal species, plant or microorganism such as bacteria, viruses, yeast or parasites. The nucleotide sequence are preferably from genomic DNA, but may be from sorted chromosomes or clones.

The vectorette units employed may be either derived from a single cleavage method and/or multiple vectorette units derived from multiple cleavage methods, which together or separately may constitute a vectorette library or plurality of vectorette libraries (as hereinafter defined).

The target nucleic acid fragments employed may be derived from a number of different sources. Thus for example the target nucleic acid fragments may be obtained from single individuals of a species of animal, plant or microorganism, for example from individuals typical of their species. The target nucleic acid fragments may also be obtained from single individuals known to be heterozygous for a given genetic locus for example a locus causing cystic fibrosis or other inherited disease. The target nucleic acid fragments may also be obtained from single individuals known to be homozygous for a given genetic locus for example cystic fibrosis or other inherited disease. The target nucleic acid fragments may also be obtained from single individuals known to be normal homozygotes for a given genetic locus for example cystic fibrosis or other inherited disease. The target nucleic acid fragments may also be obtained from groups of individuals (as opposed to single individuals) with a shared phenotype(s). The nucleic acid or tissue from each member of a group which shares a phenotype may if desired be pooled. Each group of individuals will consist of at least 2 and advantageously less than 1000, for example 50-500. Vectorette units may be prepared from the target nucleic acid fragments and the vectorette units pooled or used separately to form vectorette libraries. The shared phenotype may if desired be a disease or disease predisposition, obligate carriage of an inherited disease or a normal state with no evidence of the disease or disease predisposition.

Comparison of nucleotide sequences obtained using the method of the invention will identify any common genetic variants in the population which are "associated" with for example a given disease or disease predisposition. It will be appreciated that this extends considerably the scope for detailed analysis over and above that previously attempted using RFLP technology.

The vectorette library kit of the present invention may additionally contain vectorette primer(s), and advantageously contains one or more selected from nested first primer(s), nested vectorette primer(s) and sequencing primers. The vectorette library kit may also conveniently contain each of four different nucleoside triphosphates and an agent for polymerisation of the nucleoside triphosphates. If desired the vectorette library kit may further contain buffers for performing the invention and/or buffers for varying the magnesium, potassium and nucleoside triphosphate concentrations. These latter buffers may be desirable for determining the optimum

conditions for subsequent cycles of the method of the invention.

The present invention i) enables nucleotide sequencing to be effected by the Chemical Genetics technique either by direct DNA sequencing of short amplified, for example PCR amplified, products or from RNA transcripts, ii) enables RNA transcripts to be generated for various uses such as for example as hybridisation probes, iii) provides an alternative to the vectorettes of the Chemical Genetics technique for the construction of libraries and Chemical Genetics "steps", iv) for the amplification of sequence, other than by PCR, via RNA transcripts ; v) for the subcloning of terminal vectorette regions from amplified products ; and vi) for the subcloning of any fragment from a PCR product.

A glossary of certain terms used herein is set out below in order to assist the reader of the present specification.

The term "target nucleic acid" refers to a nucleotide sequence, generally genomic DNA, for example plant or animal genomic DNA such as human DNA or bacterial DNA. Such a "target nucleic acid" for use in the method of the present invention will normally comprise a portion (generally a small portion) of known sequence and generally a much larger portion of unknown sequence.

The term "target nucleic acid fragment" is used herein to mean a fragment of a target nucleic acid obtained by cleaving (as hereinafter defined) target nucleic acid. The term "target nucleic acid fragment" therefore is not limited to such a fragment obtained by the use of a restriction endonuclease. Furthermore such a fragment may for example comprise a portion of known sequence and generally a larger portion of unknown sequence, or the fragment may be of unknown sequence. Where the fragment is of unknown sequence, cleavage and generation of target nucleic acid fragment/vectorette units may be performed as described hereinafter. In such circumstances random DNA probes may be generated from the much larger genomic fragment by linear amplification with a "random" first primer or amplification with a random first primer plus vectorette primer. Specific amplification will generate fragments at random which can be purified and used in genetic mapping as in Cell, 51, 319-337 (1987) H. Donis-Keller et al.

The term "amplification" is used herein to refer to the replication of a nucleotide sequence and/or its complementary sequence by non-biological means and thus includes amplification by the use of a first primer alone such primer being hybridised to the first priming region of a target nucleic acid fragment/vectorette unit, primer extension being effected in the presence of appropriate nucleoside triphosphates and an agent for the polymerisation of the nucleoside triphosphates under hybridising conditions, such primer extension being followed by denaturation, this process of priming, primer extension and denaturation being repeated as many times as appropriate to achieve a desired level of amplification. The term "amplification" also includes replication by polymerase chain reaction (PCR) techniques as for example described by R.K. Saiki et al, in Science, 239, 487-491 (1987) and in U.S. Patents Nos 4,683,195 and 4,683,202 using a first primer and a vectorette primer as hereinafter defined and the expression polymerase chain reaction technique or PCR technique as used herein to refer to such techniques as described in these references.

The term "non-biological" is used herein only to exclude amplification by direct cloning and propagation of bacterial colonies. It will therefore be appreciated that the term amplification is used herein to include amplification processes such as those described in PCT Patent Publication WO 87/06270 (or Biotechnology Vol 6, October 1988), PCT Patent Publication W088/10315 or PCT Patent Publication WO 89/01050.

The term "cleaving" is used herein to refer to cleavage of a nucleic acid at a specific site, in any part of the nucleic acid fragment/vectorette unit. Such cleavage is conveniently effected using a restriction endonuclease.

In respect of the nucleic acid fragment this is preferably a 6bp cutter, having a known recognition sequence and cleavage pattern and thus the characteristic of cleaving DNA at specific sites. In this regard the position of these "specific sites" in a given target nucleic acid will not normally be known relative to the position of another known element in the target nucleic acid, but the sequence of the specific sites will be known as will their cleavage patterns. Thus the terminal sequences of the restriction fragments obtained will be known. Thus for example the restriction endonuclease EcoRI recognises the sequence :-

```
-GAATTC-             -G           +   AATTC-
-CTTAAG-  --->       -CTTAA               G-
```

and cleaves such a sequence as indicated to yield restriction fragments having the cohesive ends indicated. Since the sequence of the cohesive end is known it is possible to produce target nucleic acid fragment/vectorette units on the basis of this knowledge as described hereinafter in relation to the expression "target nucleic acid fragment/vectorette unit".

Means for cleaving a target nucleic acid at a specific site other than restriction endonucleases may be employed, but the use of a restriction endonuclease is preferred. Any convenient restriction endonuclease may

be used.

Examples of individual restriction endonucleases include those detailed in Nucleic Acids Research, Sequences Supplement, Volume 16, 1988 page r271-r313 and in "Current Protocols in Molecular Biology 1987-1988, Edited by Ausubel F.M., Brent R., Kingston R.E., Moor D.D, Smith J.A., Seidman J.G. and Struhl K., Wiley Interscience, Section 3, Table 3.1-1. Restriction endonucleases capable of producing fragments with cohesive ends such as EcoRI, Hind III and XbaI are convenient regardless of whether the cohesive ends have 5′ or 3′ overhangs. It will also be appreciated that as well as by using restriction endonucleases capable of producing fragments with either a 3′ or a 5′ overhanging cohesive end the production of target nucleic acid fragment/vectorette units is also possible using restriction endonucleases producing blunt-ended fragments in conjunction with appropriate blunt-ended vectorettes as hereinafter defined. Means for cleaving a target nucleic acid at (a) specific site(s) other than by standard restriction endonuclease digestion are known in the art and include, for example, the use of adapter-primers and Class-IIS restriction enzymes (S.C. Kim et al, Science, 240, 504-506 (1988) ; W. Szybalski, Gene, 40, 169 (1985) ; A.J. Podhajska and W. Szybalski, Gene, 40, 175 (1985)) as well as various chemical approaches (B.L. Iverson and P.B. Dervan, J. Am. Chem. Soc., 109, 1241-1243 (1987) ; G.B. Dreyer and P.B. Dervan, Proc. Natl. Acad. Sci. USA, 82, 968 (1985) ; V.V. Vlassov et al, Nucleic Acids Res., 14, 4065 (1986) ; H.E. Moser and P.B. Dervan, Science, 238, 645 (1987) ; D.R. Corey and P.G. Schultz, Science, 238, 1401 (1987) ; J.P. Sluka et al, Science, 238, 1129 (1987)).

Furthermore, it is possible to render target nucleic acid fragments blunt-ended, either by DNA polymerase mediated fill-in/repair of any 5′ overhanging cohesive ends or by removal of 3′ and/or 5′ overhangs using S1 nuclease mediated single strand digestion, irrespective of whether they are generated by restriction enzyme digestion or other, for example, chemical means. All such blunt-ended fragments may again be converted into target nucleic acid fragment/vectorette units by attachment of the appropriate blunt-ended vectorette.

The expression "first priming region" as used herein means that portion of a cleaved, for example restriction enzyme digested, target nucleic acid fragment which is of known nucleotide sequence and to which in use a first primer and if desired nested first primer(s) (as hereinafter defined) for example overlapping nested first primer(s) may hybridise. Thus for example the method of the present invention may be effected such that only one of the target nucleic acid fragments present will have a first priming region as in the Chemical Genetics Technique (so hereinbefore defined) in which the first priming region corresponds to the initiating priming region. An exception would be the use of a mixture of a plurality of different vectorette libraries as hereinafter defined. Furthermore the method of the present invention may also be effected by the use of a target nucleic acid fragment/vectorette unit having a vectorette portion at each terminus of the said unit in which case one of the vectorette portions will comprise the first priming region.

The expression "vectorette priming region" as used herein means that portion of the target nucleic acid fragment/vectorette unit which is of known nucleotide sequence as defined by the vectorette itself and to which in use the vectorette primer and, if desired nested vectorette primer(s) (as hereinafter defined) for example overlapping nested vectorette primer(s), may hybridise. The vectorette priming region is present in the strand complementary to the strand containing the initiating priming region. In this regard the "vectorette priming region" to which in use the vectorette primer and if desired nested vectorette primer(s) will hybridise may be present either in the target nucleic acid fragment/vectorette unit prepared by ligation or in a target nucleic acid fragment/vectorette unit prepared by primer extension of an initiating primer or in both. Thus it will be appreciated that the vectorette primer and if desired nested vectorette primer(s) to be used in the method of the present invention may be selected for hybridisation with a vectorette priming region which may, for example, not be generated until primer extension of an initiating primer and if desired nested initiating primer(s). A consequence of this is that, for example the vectorette itself need not be a totally self-complementary double-stranded DNA fragment.

The term "primer" as used herein refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of appropriate nucleoside triphosphates and an agent for polymerisation such as DNA polymerase in an appropriate buffer ("buffer" includes pH, ionic strength, cofactors, etc.) and at a suitable temperature.

The primer is preferably single stranded for maximum efficiency in extension, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerisation. The exact lengths of the primers will depend on many factors, including temperature and source of primer and use of the method. For example, depending on the complexity of the target sequence, the first and vectorette primers will typically contain 15-35 nucleotides, although they may contain more or fewer nucleotides. Short primer molecules generally require lower temperatures to form sufficiently stable hybrid complexes with the template.

The term "first primer" is used herein to refer to a primer capable of hybridisation with the first priming region as hereinbefore defined. In use with, for example, vectorette units prepared from total human genomic DNA, it may be preferable for the "first primer" to be longer than 15-17 nucleotides so as to avoid hybridisation and priming, at random, to sequences present in the human genome which happen by chance to match that of the first priming region.

The term "vectorette primer" is used herein to refer to a primer capable of hybridisation to the vectorette priming region of the target nucleic acid fragment/vectorette unit. The "vectorette primer" will have a nucleotide sequence such that it is capable of hybridising to an initiating primer extension product, after separation from its complement, whereby the initiating primer extension product serves as a template for synthesis of an extension product of the vectorette primer, thereby facilitating amplification. Since, in general, the method of the present invention will be effected such that only one of the target nucleic acid fragment/vectorette units has an initiating priming region, only that unit will be subjected to amplification. Those target nucleic acid fragment/vectorette units which do not have an initiating priming region will be incapable of PCR amplification because whilst the formation of a vectorette primer extension product may be possible, no initiating primer will be able to hybridise to the vectorette primer extension product since no initiating priming region will be present and thus no PCR amplification will be possible.

In use it is preferred that the synthesis of vectorette primer amplification products is dependent upon the initial synthesis of an extension product of the initiating primer. This avoids formation of large numbers of unamplifiable vectorette primer extension products which might be expected to deplete the available nucleoside triphosphates or other co-factors in the reaction mixture in a detrimental fashion.

The term "nested primer" as used herein means a primer displaced by one or more base pairs in the 3' direction away from the 5' terminus of the initiating primer or in the 3' direction away from the 5' terminus of the vectorette primer or in the 3' direction away from the 5' terminus of both such initiating primer and vectorette primer. It will be appreciated that the sequence of the nested primer or primer(s) will necessarily be selected from sequence complementary to the known initiating or vectorette priming regions or from both such regions.

The term "target nucleic acid fragment/vectorette unit" (also referred to herein as a "vectorette unit") is used herein to refer to a nucleotide sequence, for example a DNA sequence, comprising a target nucleic acid fragment and a portion of known nucleotide sequence, for example a DNA sequence, which portion in single stranded form is capable of hybridisation to a vectorette primer as hereinbefore defined. In this regard it will be appreciated that the "target nucleic acid fragment/vectorette unit" may be capable of hybridisation to a vectorette primer either by virtue of the presence in the aforesaid unit of a portion of known nucleotide sequence which sequence is substantially complementary to the sequence of the vectorette primer or by virtue of the ability of a first primer extension product, based on one strand of the aforesaid unit as template, to comprise a nucleotide sequence substantially complementary to the sequence of the vectorette primer. It will be appreciated in this regard that the "target nucleic acid fragment/vectorette unit" is such that in one strand it will have substantially the same sequence at least in part as the vectorette primer. This strand will also contain the first priming region. It will be appreciated that the term "target nucleic acid fragment/vectorette unit" also encompasses, where the context permits, a nucleotide sequence, for example a DNA sequence, comprising a target nucleic fragment having a vectorette portion at the 5' and 3' terminus of each fragment. In such a case the vectorette unit in single stranded form will have one of the vectorette portions comprising a first priming region.

The portion of known nucleotide sequence, for example, DNA sequence, may be derived from any convenient source, provided that it fulfils the above-stated requirement that in single stranded form it is capable of hybridisation to the vectorette primer. Thus for example the vectorette may be prepared separately using a DNA synthesiser and the vectorette obtained ligated to the target nucleic acid fragment(s) to obtain the target nucleic acid fragment/vectorette unit(s). In this regard the vectorette will conveniently be adapted for ligation to the nucleic acid fragment(s), for example a cohesive end on the target nucleic acid fragment(s) hybridising with a cohesive end on the vectorette to form a vectorette unit as hereinbefore defined or a blunt ended target nucleic acid fragment may be ligated to a blunt ended vectorette to form a vectorette unit as hereinbefore defined. It is not necessary however that the vectorette be pre-formed prior to ligation with the target nucleic acid fragment although this may be preferable. Thus for example the aforementioned unit may be prepared, in an appropriate case, by ligation of a single stranded DNA to the target nucleic acid fragment, for example by utilising the overhang of the cohesive end to secure a first single stranded DNA thereto and then to permit a second single stranded DNA to be ligated so as to form the desired unit.

In one embodiment of the present invention the target nucleic acid fragment/vectorette unit contains a blocking vectorette portion. The term "blocking vectorette" as used herein refers to a vectorette or to the vectorette portion of a target nucleic acid fragment/vectorette unit in which one or both free terminal bases are in modified form to prevent ligation of nucleotides thereto or to prevent primer extension for example in the presence of appropriate nucleoside triphosphates and an agent for the polymerisation of the nucleoside triphosphates under hybridising conditions. Such modifications are known per se and may for example consist of the presence of a dideoxynucleoside. Thus for example the double stranded blocking vectorette may have a 3′ terminal dideoxynucleoside such as dideoxyadenosine (ddA). Such modification may also include ribonucleosides in which the diol of the ribose is cleaved, for example with periodate. Alternatively a 3′-deoxynucleoside for example a 3′- deoxyadenosine residue may be added at the 3′-terminus using for example cordycepin triphosphate and terminal transferase. As a further alternative a 3′-amino or 3′-thio functionality may be introduced chemically at the 3′ end by methods known per se.

In a further and especially preferred embodiment of the present invention a vectorette or vectorette portion comprises two partially hybridised single stranded sequences which possess a degree of non-complementarity such that vectorette primer extension cannot be effected using such a vectorette, nucleic acid sequence in the region of the non-complementarity being capable in either its double stranded complementary form or its single stranded form, of being bound by protein.

The term "nucleoside triphosphate" is used herein to refer to the triphosphates of nucleosides present in either DNA or RNA and thus includes nucleosides which incorporate adenine, cytosine, guanine, thymine and uracil as base, the sugar moiety being deoxyribose or ribose. In general deoxyribonucleosides will be employed in combination with a DNA polymerase. It will be appreciated however that other modified bases capable of base pairing with one of the conventional bases adenine, cytosine, guanine, thymine and uracil may be employed. Such modified bases include for example 7-deazaguanine and hypoxanthine.

The term "nucleotide" as used herein can refer to nucleotides present in either DNA or RNA and thus includes nucleotides which incorporate adenine, cytosine, guanine, thymine and uracil as base, the sugar moiety being deoxyribose or ribose. It will be appreciated however that other modified bases capable of base pairing with one of the conventional bases, adenine, cytosine, guanine, thymine and uracil, may be used in the initiating primer and vectorette primer employed in the present invention. Such modified bases include for example 7-deazaguanine and hypoxanthine.

The agent for polymerisation of the nucleoside triphosphates may be any compound or system which will function to accomplish the synthesis of primer extension products, including enzymes. Suitable enzymes for this purpose include, for example, E.coli DNA Polymerase I (Richardson C.C. et al, J. Biol. Chem. 239, 222 (1964)), Klenow fragment of E.coli DNA polymerase I (Jacobsen H. et al, Eur. J. Biochem. 45, 623-627 (1974)), T4 DNA polymerase (Panet A. et al, Biochemistry 12, 5045-5050 (1973)), T7 DNA polymerase (Tabor S. and Richardson C.C., Proc. Natl. Acad. Sci. USA 84, 4767-4771 (1987)) other available DNA polymerases, reverse transcriptase, and other enzymes, including thermostable enzymes. The term "thermostable enzyme" as used herein refers to an enzyme which is relatively stable to heat and is heat resistant and catalyzes (facilitates) combination of the nucleotides in the proper manner to form the primer extension products which are complementary to each nucleic acid strand. Generally, the synthesis will be initated at the 3′ end of each primer and will proceed in the 5′ direction along the template strand, until synthesis terminates, generally producing molecules of different lengths. In the context of the current invention synthesis will generally terminate at a position determined by the target nucleic acid cleavage site resulting in molecules of the same length. There may be enzymes, including thermostable enzymes, however, which initiate synthesis at the 5′ end and proceed in the other direction, using a similar process to that described above. A preferred thermostable enzyme which may be employed in the process of the present invention is extracted and purified from Thermus aquaticus and has a molecular weight of about 86,000 - 90,000 daltons as described in European Patent Publication No. 237,362 (see also European Patent Publication No. 258,017). Thermus aquaticus strain YT1 is available without restriction from the American Type Culture Collection, 12301 Parklawn Drive, Rockville, Maryland, USA as ATCC 25,104.

It will be obvious that DNA polymerases having superior or more advantageous properties may be obtained by random mutation of clones expressing these proteins. By way of example, it would obviously be preferable to obtain a mutated version of double stranded DNA encoding Taq DNA polymerase which resulted in the expression of a protein with superior properties such as lack of a 5′-exonuclease activity. Techniques for obtaining such desired mutated versions are well known and are well within the skill of the average molecular biologist.

The term "complementary to" is used herein in relation to nucleotides to mean a nucleotide which will base pair with another specific nucleotide when incorporated into DNA or RNA. Thus deoxyadenosine triphosphate is complementary to thymidine triphosphate and deoxyguanosine triphosphate is complementary to deoxycytidine triphosphate, whilst deoxyguanosine triphosphate is not complementary to thymidine triphosphate. It is appreciated in this regard that whilst thymidine triphosphate and deoxyguanosine triphosphate may base pair under certain circumstances they are not regarded as complementary for the purposes of this specification.

The primers herein are selected to be "substantially" complementary to the different strands of each specific sequence to be extended or amplified. This means that the primers must be sufficiently complementary to hybridise with their respective strands. Therefore, the primer sequences need not reflect the exact sequence of their templates although this would normally be preferable.

The term "vectorette library" is used herein to refer to the plurality of target nucleic acid fragment/vectorette units which are obtained after cleaving a target nucleic acid at all the potential cleavage sites in respect of a given restriction endonuclease which the target nucleic acid contains and preparing target nucleic acid fragment/vectorette units from the total mixture of target nucleic acid fragments by cycles of ligation to a suitably adapted vectorette portion (and repeat cleavage if required). In general only a single vectorette unit in a given vectorette library will contain the first priming region of interest. In the case of human genomic DNA cleaved with a restriction endonuclease recognising a specific 6 bp sequence (a 6 bp cutter) the average size of the resulting target nucleic acid fragments will be 4096 bp and the target nucleic acid generates approximately $10^6$ such fragments. Hence a vectorette library containing approximately $10^6$ target nucleic acid fragment/vectorette units is obtained from human genomic DNA cleaved with a 6 bp cutter restriction endonuclease and only one such vectorette unit in that total human vectorette library will contain a given first priming region capable of initiating amplification in the presence of an initiating primer, a vectorette primer if desired, appropriate nucleoside triphosphates and an agent for polymerisation of the nucleoside triphosphates under hybridising conditions.

Different vectorette libraries may be prepared from the same target nucleic acid by cleavage with different restriction endonucleases and ligation of suitably adapted vectorette portions to generate target nucleic acid fragment/vectorette units. All available restriction endonucleases can be used in this process if desired and in the limit a vectorette portion can be ligated to target nucleic acid fragments at every restriction enzyme recognition site in the target nucleic acid. This feature is not always desirable as ideally the first primer region of interest in any given vectorette library will be separated by 100 bp or more from the attachment point of the vectorette portion. This is because first primer extension products or first primer/vectorette primer amplification products smaller than this generate so little sequence information in the practice of the invention as to be of little value for the efficient sequencing of long nucleotide sequences. Furthermore the nucleotide sequence of such small products will be contained within the products obtained using a vectorette library in which the first primer is further from the vectorette portion attachment site. The use of a plurality of different vectorette libraries with a particular first primer allows identification of those libraries wherein the extension or amplification products are of a convenient size for sequencing. For example it may be particularly convenient to select first primer extension or amplification products of approximately 200 bp, 400 bp, 600 bp, 800 bp, 1000 bp and so on obtained from particular vectorette libraries with a given first primer. Sequencing of such products, from the vectorette libraries in which they happen to occur for a given first primer, using a vectorette or nested vectorette sequencing primer and methods known per se is likely to generate overlapping sequence data for a large region to the 3'-side of the first primer. The amount of sequence data generated in one round of analysis of a plurality of vectorette libraries with a given first primer is only limited by the size of first primer extension or amplification products which can be obtained in practice and/or by the distance (from the first primer region) to the most remote restriction endonuclease site represented in the plurality of vectorette libraries.

In an advantageous embodiment of the method of the present invention, the target nucleic acid fragment/vectorette units are prepared from target nucleic acid fragments by ligation such that the vectorette cannot be cleaved from the target nucleic acid fragment/vectorette unit formed by the same agent used to cleave the target nucleic acid to yield target nucleic acid fragments. It is especially preferred that the target nucleic acid is cleaved with a restriction endonuclease to yield a target nucleic acid fragment for ligation to a vectorette, the sequence of the vectorette being selected such that the restriction endonuclease recognition sequence of the said restriction endonuclease is absent in the target nucleic acid fragment/vectorette unit, for example in the case of EcoR1 described

```
-GAATTC          -G          + AATTX-
-CTTAAG  --->    -CTTAA           Y-   - Vectorette

           Target nucleic acid
                fragment
```

above where X is any nucleoside other than C and Y is its complementary nucleoside.

In order that the present invention may be more fully understood it is described hereinafter, by way of example, with reference to the accompanying drawings, in which :-

Figure 1(a) shows a blocking vectorette portion for use in the Chemical Genetics technique (as hereinbefore defined) and Figure 1(b) shows a schematic representation of the application of the Chemical Genetics technique to the blocking vectorette portions of Figure 1(a). Figure 1(a) and (b) also show the relative position of the nucleic acid sequence which in either its double stranded complementary form or its single stranded form is capable of being bound by protein according to the present invention.

Figure 2(a) shows a non-complementary vectorette portion for use in the Chemical Genetics technique (as hereinbefore defined) and Figure 2(b) shows a schematic representation of the application of the present invention to the non-complementary vectorette portions of Figure 2(a). Figures 2(a) and 2(b) also show the relative position of the nucleic acid sequence which in either its double stranded complementary form or its single stranded form is capable of being bound by protein according to the present invention.

Figure 3 shows the structural design of vectorettes and primers according to the present invention ;

Figure 4 shows the relative locations of M0MP.4.XbaI, M0MP.6. Vectorette in respect of the MOMP (major outer membrane protein) region of C.trachomatis (European Patent No.198 083) and of CRP.9.8 in respect of a cysteine-rich outer membrane protein (CRP) locus (Clarke, I.N. et al (1988) Gene 71, 307-314) ;

Figure 5 shows a comparison by agarose gel analysis of amplified fragments obtained by PCR according to the Chemical Genetics technique as hereinbefore defined and according to the method of the present invention;

Figure 6 shows the bands generated by performing PCR with primers 6 and 1 on various vectorette libraries ;

Figure 7 shows amplification by PCR of the BamHI DEVIATS sub-fragments from M0MP.6.Vectorette (ClaI) according to the method of the present invention (The term "DEVIATS" as used hereinafter means double ended vectorettes incorporating alternative transcription sites) ;

Figure 8 shows amplification by PCR of Chlamydia-infected and uninfected McCoy cells using primer 6 in combination with two primers derived from the sequences of the BamHI DEVIATS sub fragment of the 6-ClaI vectorette step ;

Figure 9 shows an autoradiograph of a Southern blot of restriction enzyme digests of chlamydial DNA hybridised with a labelled RNA transcript from 9-ClaI DEVIATS ;

Figure 10 shows an autoradiograph of a Southern blot of restriction enzyme digests of chlamydial DNA hybridised with a labelled RNA transcript from 8-ClaI DEVIATS ;

Figure 11 shows amplification by PCR using specific primers with EcoRI Vectorette and DEVIATS chlamydial libraries ;

Figure 12 shows RNA transcript generation following single-cycle primer extension on M13mp18-XbaI DEVIATS ; and

Figure 13 shows the sequence derived from the BamHI DEVIATS sub-fragments of MOMP6. obtained according to Example 6.

Figure 14 shows digestion and religation of a restriction site in the Vectorette 2 sequence.

More particularly Figure 1(a) shows a blocking vectorette having a cohesive end (1) for annealing to complementary ends produced in cleaved target DNA. The 3′end of the upper strand is modified (x) such that 5′--3′ extension from that end cannot be carried out using any of the known DNA polymerases, for example Klenow fragment, T7 DNA polymerase (Sequenase) or Taq DNA polymerase. This modified terminus may involve the use of a modified base such as a dideoxynucleoside or 3′-deoxynucleoside (for example cordycepin). The 3′-sugar residue may, if desired, be modified by any chemical methods known per se. The 3′ terminus of the lower strand and the 5′ terminus of the upper strand of the blocking vectorette are designed to anneal to complementary ends of the cleaved target DNA, with the proviso that following ligation of the vectorette and the target DNA the recognition sequence for the restriction endonuclease used to cleave the target DNA originally is destroyed so that further cleavage at this site is no longer possible.

A blocking vectorette for use with the present invention would additionally comprise a nucleic acid sequence which in either its double stranded complementary form or its single stranded form is capable of being bound by protein, such sequence being present in the region indicated by 2 in Figure 1(a).

Figure 1(b) shows a schematic representation of the application of the present invention to the vectorette portion of Figure 1(a). Genomic target DNA is digested to completion with a single restriction endonuclease to produce fragments as shown in (i). The blocking vectorette portions [3 in Figure 1(b)(i)] are ligated onto the ends of every cleaved genomic target DNA fragment (ii) in the presence of DNA ligase. Amplification is then carried out after denaturation using the primers of known nucleotide sequence x and y in the presence of for example Taq polymerase.

Thus in Figure 1(b) (iii), x represents the first primer and possesses the same or at least substantially the same sequence as the region marked FP (first primer) in strand 1. x is capable of hybridising to the first priming region (FPR) in strand 2. Strand 2 which contains the first priming region (FPR) also contains a portion of nucleotide sequence which is the same or at least substantially the same as the nucleotide sequence of the vectorette primer (VP). In use, primer extension of x yields a strand which contains a vectorette priming region

(VPR) represented by the primer y. Single strand 1 does not contain a vectorette priming region itself and cannot be subject to primer extension because of the presence of the polymerisation blocking moiety and strand 2 also has no vectorette priming region, no primer extension of a vectorette primer can take place until a vectorette priming region is created by primer extension of the initiating primer.

It will therefore be appreciated that in the first amplification cycle only the primer x can produce an extension product extending up to the end of strand 2 of the blocking vectorette (iii). Primer y is redundant in the first cycle since there is no complementary sequence for it to hybridise to and produce extension products. The sequence of primer y is exactly the same as bases from within the 5′ portion of strand 2, which portion is single stranded in vectorette form. In the second cycle and thereafter (v and vi) the primer y can hybridise to the extension product of primer x and complementary synthesis of this extended product (iv) may proceed. Thus primer y cannot hybridise to any of the ligation products shown in (iii). It can only hybridise to an essentially completely extended product of primer x which is the known nucleotide sequence in the locus of interest. Thus only an amplification product containing primer x, unknown nucleotide sequence z and primer y is produced. The nucleotide sequence of z can be determined from both ends by using primer x and y as sequencing primers. Alternatively "nested" sequencing primers x′ and y′ may be prepared. These will contain sequence 3′ of primer x or 3′ of primer y in Figure 1(b).

Figure 2(a) illustrates a non-complementary vectorette in which the vertical lines indicate hybridisation. The cohesive end (1) is as described in relation to Figure 1(a). The region (5) is the non-complementary region of the vectorette, the degree of non-complementarity being such that a primer capable of hybridisation to strand 3 in region 5 will not hybridise to strand 4 in the same region and under the same conditions. A non-complementary vectorette for use with the present invention would comprise in region 5 a nucleic acid sequence which in either its double stranded complementary form or its single stranded form is capable of being bound by protein. Such a sequence may be present in strand 3 or strand 4 or different such sequences may be present in each of strands 3 and 4.

Figure 2(b) illustrates the application of the method of the present invention to the non-complementary vectorettes of Figure 2(a).

Figure 1 illustrates the method of the present invention by reference to a target nucleic acid fragment/vectorette unit in which the vectorette portion contains a polymerisation blocking moiety. Similar considerations apply where the vectorette portion comprises at least a region of non-complementarity as hereinbefore described and as illustrated by way of example in Figure 2(a). Thus in Figure 2(b) a vectorette for use with the present invention (A) as depicted in Figure 2(a) is ligated to a nucleic acid fragment/vectorette (B) such as would be obtained using the Chemical Genetic technique. B consists of unknown nucleotide sequence (Z) ligated to a Chemical Genetics vectorette (6), the region 7 being double stranded complementary sequence as opposed to the double stranded region 5 in (A). The region (7) serves as the first priming region and x is the first primer. Primer extension of x as shown in Figure 2(b)(iii) results in an extension product to which primer y may hybridise in order to amplify the unknown sequence (z) by PCR> Because of the non-complementarity between the strands 3 and 4 in region 5, the primer y cannot hybridise to either strand 3 or strand 4, but only to the extension product of primer x.

In Figure 2(a) and 2(b) the vectorette (A) is shown as having one cohesive end and one blunt end. It will be appreciated that each end of the vectorette may have a cohesive end as shown for example in Figure 3, each end for example being adapted for ligation to nucleic acid strands having different cleavage sites(for example EcoRI and XbaI cleavage sites). If desired the region of non-complementarity may comprise one strand capable of being bound by one protein (for example an RNA polymerase such as SP6 RNA polymerase), the other strand being capable of being bound by a different protein (for example an RNA polymerase such as T7 RNA polymerase). Thus in Figure 3 EcoRI ligation might result in use of T7 RNA polymerase binding and XbaI ligation might result in the use of SP6 RNA polymerase binding.

According to a further feature of the present invention we provide a vectorette comprising double stranded nucleic acid having a region of non-complementarity, this region having in a first strand a nucleic acid sequence which in either its double stranded complementary form or in its single stranded form is capable of being bound by one protein (for example a DNA or RNA polymerase such as SP6 RNA polymerase) and this region having in a second strand a nucleic acid sequence which in either its double stranded complementary form or in its single stranded form is capable of being bound by a protein different from that which is capable of binding the nucleic acid sequence in the first strand (for example a different DNA or RNA polymerase such as T7 RNA polymerase). The region of non-complementarity is desirably sandwiched between regions of complementarity. The vectorette may have blunt ends or preferably cohesive ends or one blunt and one cohesive end.

The present invention will now be illustrated, but not limited by reference to the following Examples in which the following oligodeoxynucleotides detailed below were used, each nucleotide sequence stated herein being read in the conventional 5′ – 3′ sense unless otherwise stated :-

A) Vectorette PCR Primer

Oligonucleotide 1

CGAATCGTAACCGTTCGTACGAGAATCGCT

B) Vectorette sequencing primer

Oligonucleotide 2

AGAATCGCTGTCCTCTCCTT
This is a lengthened vectorette sequencing primer having an additional five nucleotides at the 5' end.

Oligonucleotide 2A

CGCTGTCCTCTCCTT
This is the vectorette sequencing primer

C) Oligonucleotides specific for the MOMP locus of Chlamydia trachomatis (see European Patent Publication No 192 033 for confirmation of the positions of the oligonucleotides) :-

Oligonucleotide 3

CTGCTCACGTAAATGCACAATTCCG - positions 2441 to 2465

Oligonucleotide 4

TGAAATCGGTATTAGTGTTTGCCGC - positions 1301 to 1325

Oligonucleotide 5

GTGCATTTACGTGAGCAGCTCTCTC - positions 2458 to 2434

Oligonucleotide 6

CCTGAAGGGCGCACAGTAGCTGAT - positions 131 to 108

Oligonucleotide 7

CAAGGCATAACGTGTTGATTGGTG - approximately 200 bp downstream of position 3133 (terminal EcoRI)

D) Oligonucleotides specific for the CRP locus of Chlamydia tranchomatis (see Clarke I.N. et al (1988) Gene 71, 307-314) :-

Oliogonucleotide 8

GGGTCTGATCCACCAGACTATTTCT - positions 2402 - 2378

Oligonucleotide 9

CTTCCGATACATTGACTGTTCCAGT - positions 1722 to 1746

E) Oligonucleotide specific for 23s rRNA (see European Patent Publication No. 272009, page 42)

Oligonucleotide 10

CGTTCTCATCGCTCTACGGAC - sequence 7, about 1180 bp into the 23s rRNA gene.

F) Oligonucleotides specific for M13mp18 DNA (sequence obtainable from Genbank Database)

Oligonucleotide 11

TTGACGTTGGAGTCCAGGTTCTTTA - positions 5757 to 5781

Oligonucleotide 12

GAGTTCTTCTACTCAGGCAAGTGAT - positions 5251 to 5275

Oligonucleotide 13

ATTCGCCTCTGCGCGATTTTGTAAC - positions 4302 to 4326

Oligonucleotide 14

TGTGAATATCAAGGCCAATCGTCTG - positions 2233 to 2257

G) The following oligonucleotides were used to construct double-ended vectorettes incorporating alternative transcription sites (DEVIATS) as shown in figure 3 :

Oligonucleotide 15

AATTGCAGGAGAACCCATTTAGGTGACACTATAGAATACGGCG

Oligonucleotide 16

CTAGCGCCGTATTCTAATACGACTCACTATAGGGTTCTCCTGC

Oligonucleotide 17

GATCGCAGGAGAACCCATTTAGGTGACACTATAGAATACGGCG

Oligonucleotide 18

CGCGCCGTATTCTAATACGACTCACTATAGGGTTCTCCTGC

H) Oligonucleotides which serve as DEVIATS primers for amplification.

a) for use with DNA digested with the restriction endonucleases XbaI and ClaI :-

Oligonucleotide 19

ACCCATTTAGGTGACAC

b) for use with DNA digested with the restriction endonucleases EcoRI, Sau3AI and BamHI :-

Oligonucleotide 20

TTCTAATACGACTCACT

The DEVIATS were constructed by mixing equimolar amounts of two oligonucleotides (either oligonucleotides 15 and 16 or oligonucleotides 17 and 18 as shown in Figure 3), heating the mixture to 94°C for 5 minutes and allowing to cool slowly to room temperature to anneal the two strands.

I) Oligonucleotides which serve as vectorettes

a) for use with DNA digested with the restriction endonuclease BamHI :-

Oligonucleotide 21

GATCGAAGGAGAGGACGCTGTCTGTCGAAGGTAAAGGAACGGAGGAGAGAAGGGAGAG

Oligonucleotide 22

CTCTCCCTTCTCGAATCGTAACCGTTCGTACGAGAATCGCTGTCCTCTCCTTC

b) for use with DNA digested with the restriction endonuclease ClaI :-

Oligonucleotide 22 (as hereinbefore defined) ; and

Oligonucleotide 23

CGGAAGGAGAGGACGCTGTCTGTCGAAGGTAAGGAACGGAGGAGAGAAGGGAGAG

c) for use with DNA digested with the restriction endonuclease HindIII :-

Oligonucleotide 22 (as hereinbefore defined) ; and

Oligonucleotide 24

AGCTGAAGGAGAGGACGCTGTCTGTCGAAGGTAAGGAACGGAGGAGAGAAGGGAGAG

d) for use with DNA digested with the restriction endonuclease XbaI :-

Oligonucleotide 22 (as hereinbefore defined) ; and

Oligonucleotide 25

CTAGGAAGGAGAGGACGCTGTCTGTCGAAGGTAAGGAACGGAGGAGAGAAGGGAGAG

e) for use with DNA digested with the restriction endonuclease SalI :-

Oligonucleotide 22 (as hereinbefore defined) ; and

Oligonucleotide 26

TCGAGAAGGAGAGGACGCTGTCTGTCGAAGGTAAGGAACGGAGGAGAGAAGGGAGAG

f) for use with DNA digested with the restriction endonuclease PstI :-

Oligonucleotide 27

AAAGGAGAGGACGCTGTCTGTCGAAGGTAAGGAACGGAGGAGAGAAGGGAGAG

Oligonucleotide 28

3' ACGTTTTCCTCTCCTGTCGCTAAGAGCATGCTTGCCAATGCTAAGCTCTTCCCTCTC 5'

g) for use with DNA digested with the restriction endonuclease SacI :-

Oligonucleotide 27 (as hereinbefore defined) and

Oligonucleotide 29

3' TCGATTTCCTCTCCTGTCGCTAAGAGCATGCTTGCCAATGCTAAGCTCTTCCCTCTC 5'

Oligonucleotide 30

CGGCAGGAGA ACCCCAGAGT TCGGGATGAT GCTTGCTAGA TGTAGAATAC GGCG

Oligonucleotide 31

CGCCGTATTC TAGTACTTCG AACCTTAAGC CCTAGGTGCA GGGTTCTTCC TGC

Oligonucleotides 30 & 31 are used to construct the cleaved Vectorette 2 units for use with DNA digested with Cla I

Oligonucleotide 32

CTTCGAACCT TAAGCCCTAG GTGCA

primer for use with Vectorette 2 unit in amplification

Oligonucleotide 33

GAATTGACGG TGAATGTACA TAACG

primer specific for Chlamydia 16S ribosomal RNA gene 5' flanking region

All oligonucleotides were prepared on an Applied Biosystems 380B DNA Synthesiser using cyanoethyl phosphoramidite chemistry and following the manufacturer's instructions. Alternatively, the oligonucleotides may be prepared by manual methods as described by Atkinson and Smith in 'Oligonucleotide Synthesis, a Practical Approach' (M.J. Gait Editor, IRL Press, Oxford, Washington DC pp 35-81).

Reference Example 1

Preparation of Chlamydial DNA

Chlamydia trachomatis serovar L2 was cultured in McCoy cells and elementary bodies harvested and purified using the method described in Caldwell H.D. et al (1981) Infect. Immunol. 31, 1161-1176. An additional step involved the treatment of the intact elementary bodies with DNase (5µg/ml) prior to the final wash step to reduce contamination with McCoy cell DNA.

To obtain purified C. trachomatis DNA, the elementary bodies were treated with 1% SDS, 5mM DTT at 65°C for 20 minutes, 10 µg/ml DNase- free RNase at 37°C for 20 minutes and 20 µg/ml proteinase K at 50°C overnight. The Chlamydial DNA was phenol extracted, ethanol precipitated, redissolved in 10mM Tris.HCl (pH7.6), 1mM EDTA aliquotted and stored at -20°C.

Reference Example 2

Preparation of Vectorette and DEVIATS Libraries of Chlamydial DNA

Vectorette chlamydial DNA libraries were made for eight restriction enzymes :

EcoRI
BamHI
ClaI
HindIII
XbaI
SalI
PstI
SacI

For each library, 150ng Chlamydial DNA was digested with 1µl high concentration enzyme (40-70 units) in a 50µl volume at 37°C for 1 hour. The restricted DNA was ligated to 200 fmol of Vectorette oligonucleotides in the presence of the appropriate buffer for each restriction enzyme, by the addition of a solution containing 2mM ATP, 2mM DTT and 1 unit T4 DNA ligase. Ligation conditions were three cycles of 20°C/60 mins and 37°C/30 mins. For use, aliquots of the library were diluted 1 :10 in water and stored at -20°C. An EcoRI DEVIATS Chlamydial library was prepared using identical conditions and materials. In addition, preparations of Chlamydial DNA digested with the same eight restriction enzymes were electrophoresed in agarose gels and Southern blots prepared for hybridisation with labelled fragments for restriction analysis.

Reference Example 3

General Conditions used for amplification by PCR

PCR reactions were in 100µl volumes overlayed with 50µl light mineral oil (Sigma). The final reaction mixture contained 67mM Tris. HCl (pH8.5), 16.6mM $(NH_4)_2SO_4$, 2mM $MgCl_2$, 10mM Beta-Mercaptoethanol, 170µgml $^{-1}$ bovine serum albumin, 40µM of each deoxynucleotide (dATP, dGTP, dCTP, TTP), 1µM of each oligonucleotide primer, and 1-10µl of target DNA library solution (usually less than 10ng). Reaction mixtures as above were heated to 94°C, 3-5 min before addition of 1 unit Taq DNA Polymerase (Perkin-Elmer/Cetus). Thereafter, 35 cycles of 94°C, 0.7min ; 64°C, 0.7 min ; 72°C, 1.5min per cycle were performed on a heating block (TECHNE PHC-1 Programmable Dri-block).

Example 1

MOMP.4.XbaI (see Figure 4 from the XbaI Vectorette library) was amplified by PCR using oligonucleotide 4 and vectorette primer 1 (as hereinbefore defined) and the general PCR conditions described in Reference Example 3. An aliquot (10µl) from the amplification reaction was on an agarose gel for analysis of the result (see Lane 1 of Figure 5). The remainder of the amplification product was precipitated with ethanol, washed, dried and resuspended in 20µl of sterile distilled water. The amplified product (5µl) was then separately digested in a volume of 50µl with EcoRI and Sau3AI at 37°C for 60 minutes. The Sau3AI digest was then heat inactivated at 65°C for 10 minutes. The EcoRI restriction enzyme was not inactivated. The fragments obtained were examined on an agarose gel (see Lanes 2 and 3 of Figure 5).

If desirable, fragments can be excised from low melting [Nusieve (FMC)] agarose gels either before or after the restriction enzyme digestion. In either case, the gel slice is melted at 70°C for 10 minutes then equilibrated at 37°C ; aliquots may be removed as required for ligation.

The digested fragments were subjected to ligation. Thus to the digested fragments, were added ATP and DTT (to 2mM final concentration each), appropriate DEVIATS (to an approximate 1.5 fold minimum molar excess), and T4 DNA ligase (1 unit). Oligonucleotides 15 and 16 were used in respect of the EcoRI digest and oligonucleotide 17 and 18 were used in respect of the Sau3AI digest. The ligation EcoRI mix was then incubated for 3 cycles of 20°C for 60 minutes and 37°C for 30 minutes per cycle ; the Sau3AI digest as incubated at 20°C, 4.5 hours for ligation to DEVIATS. Following ligation, the volume was adjusted to 100µl with sterile distilled water and the ligated samples stored at -20°C.

For PCR amplification of DEVIATS sub-fragments, 1µl of the ligated material is used per reaction. DEVIATS PCR primer 20 together with oligonucleotide 2 (vectorette primer) were used and the annealing step of the PCR was reduced to 45°C for 0.7 minutes from 64°C for 0.7 minutes. PCR products (10µl of each PCR product were analysed on an agarose gel as shown in Figure 5 in which the full-length PCR product (MOMP.4.XbaI, about 1100bp) generated with primers 1 and 4 is shown in lane 1. A small number of non-specific bands can also be seen. Digestion with EcoRI gave 4 fragments (lane 2) of 123, 131, 361 and 498bp ; the 361 bp fragment includes the XbaI - terminal Vectorette. Digestion with Sau3AI gave 7 fragments ranging in size from 9 to 352 bp (lane 3), the 352 bp fragment includes the ClaI - terminal Vectorette. PCR amplification following ligation of DEVIATS to these digests, and using DEVIATS and Vectorette primers, gave specific amplification of the Vectorette terminal fragments (lanes 4 and 5), these being the fragments distal to the end of MOMP 4.XbaI generated by the initial sequence-specific primer 4.

Thus lanes 1 to 6 in Figure 5 relate to MOMP.4.XbaI and are as follows :-

Lane 1 : full length PCR product
2 : EcoRI fragments
3 : Sau3AI fragments
4 : EcoRI-DEVIATS - PCR with primer 4
5 : Sau3AI-DEVIATS - PCR with primer 4
6 : 0x174 HaeIII size marker

Example 2

CRP.9.8 (see Figure 4) from a chlamydial DNA preparation (see Reference Example 1) was amplified by PCR using oligonucleotides 9 and 8 (as hereinbefore defined) and the general PCR conditions described in Reference Example 3. An aliquot (10µl) from the amplification reaction was run on an agarose gel for analysis of the result (see lane 7 of Figure 5). The remainder of the amplification product was precipitated with ethanol, washed, dried and resuspended in 20µl of sterile distilled water. Aliquots of these amplified fragments (5µl) were then separately digested in a volume of 50µl with XbaI or ClaI at 37°C for 60 minutes. The restriction enzymes were not inactivated. The digested fragments were then subjected to ligation. Thus to the digested fragments were added ATP and DTT (to 2mM final concentration each), appropriate DEVIATS (to an approximate 1.5 fold minimum molar excess), and T4 DNA ligase (1 unit). Oligonucleotide 15 and 16 were used in respect of the XbaI digest and oligonucleotides 17 and 18 were used in respect of the ClaI digest. The ligation/restriction enzyme mixes were then incubated for 3 cycles of 20°C for 60 minutes and 37°C for 30 minutes per cycle. Following ligation, the volume was adjusted to 100µl with sterile distilled water and ligated samples stored at -20°C.

For PCR amplification of DEVIATS sub-fragments, 1µl of the ligated material is used per reaction. DEVIATS PCR primer 19 together with oligonucleotide 2 (vectorette primer) were used and the annealing step of the PCR was reduced to 45°C for 0.7 minutes from 64°C for 0.7 minutes. PCR products (10µl of each PCR product) were analysed on agarose gels as shown in Figure 5 in which the 680 bp full-length PCR product is shown in lane 7. Bigestion with XbaI gives fragments of 30, 160 and 490 bp (lane 8) ; the 160 bp fragment has oligonucleotide 9 at its 5′ end. Digestion with ClaI gives fragments of 68, 113 and 499 bp (lane 9) ; the 68 bp fragment has oligonucleotide 9 at its 5′ end.

PCR amplification following DEVIATS ligation to these digests, and using primer 9 (specific) and 19 (DEVIATS), gave a specific amplification of the primer 9 terminal fragments (each now increased in size by 30bp due to amplification also of a portion of the DEVIATS ; lanes 10 and 11).

Thus lanes 6 to 11 in Figure 5 are as follows :-

Lane 6 : 0x174 Hae III size marker
7 : full length PCR product (680bp)
8 : XbaI fragments
9 : ClaI fragments
10 : XbaI-DEVIATS PCR with primer 9
11 : ClaI-DEVIATS PCR with primer 9

Example 3

Uncharacterised vectorette fragments upstream of the MOMP locus (see Figure 4) from each of the vectorette libraries (BamHI, ClaI, EcoRI, HindIII, PstI, SacI, SalI and XbaI vectorette libraries) were amplified by PCR using oligonucleotides 1 and 6 (as hereinbefore defined) and the general PCR conditions described in Reference Example 3. The oligonucleotides constituting the vectorettes are oligonucleotides 21-29 as hereinbefore defined.

Figure 6 shows the bands generated by performing such a PCR amplification in which

Track No : 1 and 10 : 0x174, Hae III size markers
2 EcoRI vectorette library
3 BamHI vectorette library
4 ClaI vectorette library
5 HindIII vectorette library
6 XbaI vectorette library
7 SalI vectorette library
8 PstI vectorette library
9 SacI vectorette library

Products were produced with the following libraries : BamHI (120 bp) ; ClaI (1300 bp) ; HindIII (200 bp) ; PstI (150 bp) ; SacI (500 bp). The fragment from the ClaI PCR was selected for further study.

This fragment was digested with BamHI and ligated to the appropriate DEVIATS. These being formed by the oligonucleotides 2 and 20 resulted in a product of approximately 580 bp. The ligation products were reamplified by asymmetric PCR (50 pmole primer 20 and 0.5 pmole primer 2) and by standard PCR, with one of the primers kinased in order to prepare material for sequencing after lambda exonuclease digestion (see Figure 7 and below). In Figure 7 the tracks are as follows :-

Track No : 1 and 7, 0x174, Hae III size markers
2 APCr (50 pmole primer 20 ; 0.5 pmole primer 2)
3 PCR (100 pmole primer 20-p ; 100 pmoles primer 2)
4 PCR (100 pmole primer 20-p ; 100 pmoles primer 2A)
5 PCR (100 pmole primer 20 ; 100 pmoles primer 2)
6 PCR (100 pmole primer 20 ; 100 pmoles primer 2A)

(wherein APCr means asymmetric PCR and -P means that the primer is phosphorylated at the 5′- end)

Sequencing was performed as described above. The resulting consensus sequence was used to design two further PCR primers which were used in PCR reactions with primer 6, with material prepared from Chlamydia - infected and uninfected McCoy cells, as the target. The results are shown in Figure 8 confirming that the sequence obtained is derived from Chlamydia trachomatis $L_2$ upstream of primer 6.

Figure 8 shows the products of a PCR reaction of Chlamydia-infected and uninfected McCoy cells using oligonucleotide 6 in combination with two primers derived from the sequences of the BamHI - DEVIATS subfragment of the primer 6 - Cla I vectorette step ; in which

Track No : 1 and 8 0x174, HaeIII size markers
2 PCR : primers 1 and 6 ; McCoy cells infected with

C.trachomatis $L_2$

4 PCR : primers 2 and 6 ; McCoy cells infected with C.trachomatis $L_2$

5 PCR : primers 1 and 6 ; unifected McCoy cells
7 PCR : primers 2 and 6 ; unifected McCoy cells

Example 4

Sequencing of the EcoRI and Sau3AI DEVIATS sub-fragments of MOMP. 4. XbaI (see Figure 4)

a) Preparation of DNA template

Sub-fragments of DNA (see Figure 4) for sequencing were prepared both by asymmetric PCR and PCR followed by Lambda Exonuclease selective digestion of one 5′-phosphorylated strand.

Asymmetric PCR's were carried out following the method of Innis et al (1988) Proc. Natl. Acad. Sci. USA 85, 9436-9440. Briefly, the DEVIATS primer is in excess (50 pmol per reaction) over the other primer used (specific primer or vectorette primer at 0.5 pmol/reaction). Other PCR conditions are as normal. Following the reaction, nucleotides, primers and Taq polymerase are removed by application of the mix to a Sephadex G-50 column. Template for sequencing was also prepared following the method of Higuchi and Ochman (1989) Nucleic Acids Research 17, 5865. Briefly, one of the primers is prepared with a 5′ phosphate group. PCR is carried out as before. The product is purified by phenol/chloroform extraction and, after ethanol precipitation, is resuspended in 67mM glycine, 10mM magnesium chloride (pH9.0) and treated with Lambda exonuclease for 15 minutes at 37°C. Single-stranded DNA is purified by phenol/chloroform extraction followed by ethanol precipitation and resuspension water.

b) The DNA template thus produced was sequenced by dideoxy sequencing (Sanger, F. et al Proc. Natl. Acad. Sci. USA 74, 5463-5467) using Taq DNA polymerase (United States Biochemical Product no. 71060) The sequencing primer was labelled with gamma $^{32}P$ dATP.

c) Full length MOMP.4.XbaI (see Figures 4 and 5) is approximately 1100 bp long and gave no readable sequence on standard direct sequencing (Newton C.R. et al (1988), Nucleic Acids Research, 16, 8233-8243). However each of the EcoRI and Sau3AI DEVIATS sub-fragments (see Figure 4) gave readable sequence as set out in European Patent Publication No 192,033.

Example 5

Sequencing of the XbaI -DEVIATS sub-fragments of CRP.9.8 (see Figure 4)

The DNA template was produced and sequenced as described in Example 4(a) and (b).

Full length CRP.9.8 gave non-continuous readable sequence of approximately 200bp in tots on standard direct sequencing (Newton et al 1988 Nucleic Acids Research, 16 8233-8243). However, present within this sequence were equivocal regions, probably due to the presence of double-stranded template. In comparison, the XbaI-DEVIATS sub-fragments of CRP.9.8 (Figure 4) gave continuous readable sequence along its whole length, but without any occurrence of multiple banding. The sequence obtained gave a 100% match with published sequence (see Clarke I. N (1988), Gene 71, 307-314).

Example 6

Sequencing of the BamHI DEVIATS sub-fragments of MOMP 6 (see Figure 4)

MOMP.6. Vectorette (see Example 3) could not be produced in sufficient amounts to act as a template for sequencing. However, using the BamHI DEVIATS sub-fragments (Figure 4), and combining results from asymmetric PCR and Lambda Exonuclease-treated PCR product (see Example 4), over 400 bp could be easily sequenced, this sequence being depicted in Figure 13.

Example 7

Sequencing of DEVIATS RNA transcripts by the use of EcoRI and Sau3AI DEVIATS sub-fragments from MOMP.4.XbaI

(a) Preparation of RNA templates

RNA was transcribed from DEVIATS sub-fragments after PCR using either SP6 or T7 RNA polymerase (depending upon orientation of the DEVIATS). RNA was purified by phenol/chloroform extraction, ethanol precipitation and resuspension in diethylpyrocarbonate (DEPC) treated water.

(b) RNA sequencing

RNA transcripts were sequenced using AMV (Avian myeloblastoma virus) reverse transcriptase and the dideoxy sequencing method (Sanger F. et al, Proc.Natl.Acad.Sci USA 74, 5463-5467)

(c) Sequencing of DEVIATS RNA Transcripts

Using DEVIATS sub-fragments from MOMP.4.XbaI, approximately 140 bp were sequenced from RNA transcripts. The sequence gave a 100% match with both previously published sequence (see European patent publication No.192033) and sequence obtained by direct DNA sequencing of PCR products (see Examples 4-6).

Example 8

Preparation and Use of DEVIATS derived RNA transcripts as Probes

(a) Preparation of RNA transcripts

Sub fragments (a) primer 9 - ClaI DEVIATS
(b) primer 8 - ClaI DEVIATS
were amplified by PCR using specific primers 9 and 8 respectively plus DEVIATS primer 19 in a 100μl reaction containing 1μl target DNA solution as defined above. The PCR products obtained were purified on a molecular sieve (Sephadex G50 spun column), ethanol precipitated, redissolved in 10μl DEPC-treated water and stored at -20°C until use.

Labelled RNA transcripts of the two DEVIATS fragments were prepared with alpha $^{32}$P-ATP (Amersham) using SP6 Polymerase. As a control, RNA transcripts were made in parallel from plasmid fragments containing active SP6 promoter sequences (Melton et al (1984) Nucleic Acids Research 12, 7035-56).

The prepared transcripts were purified by the use of molecular sieves (for example Sephadex G50 spun column) to remove unincorporated label ; aliquots were collected before and after purification and radioactivity measured by Cherenkov counting and percentage incorporation calculated. The RNA transcripts of the primer 9 ClaI and primer 8 ClaI DEVIATS fragments were divided into two portions and tested separately for confirmation of RNA synthesis and for utility as RNA probes (see (c) below).

(b) Confirmation of Synthesis of RNA

To confirm that alpha $^{32}$P-labelled nucleotides were incorporated into RNA transcripts, TCA precipitation experiments were carried out on (a) untreated transcripts, (b) DNase treated transcripts (c) RNase treated transcripts. The method of Little and Jackson (1987) In : DNA Cloning Volume III pp1-42. Glover A.M. (Ed). IRL Press, Oxford was employed. Each labelled RNA transcript sample primer 9-ClaI, primer8-ClaI, and control) was split into three equal aliquots. To the first aliquot was added 1 unit RNase-free DNase ; to the second was added 10μg DNAse-free RNase ; the third aliquot served as untreated control and all three were incubated at 37°C for 30 minutes. Each aliquot was absorbed onto duplicate filter paper (Whatman DE81), allowed to dry and radioactivity measured by Cherenkov counting. The filters were washed nine times in 20mls TCA solution before drying and recounting. The percentage of TCA precipitable counts remaining after each treatment was calculated (see Table below).

(c) Confirmation of Synthesis of RNA Transcripts

TCA precipitation of RNA Polymerase reaction products, following DNase and RNase treatment, confirmed that the labelled fragments produced were RNA (see Table below)

TABLE

Confirmation of DEVIATS mediated RNA synthesis by DNAse and RNAse treatments of Transcription Products

| TCA Precipitable Counts in RNA transcription reaction | primer 9 -ClaI | primer 8 -ClaI | Control |
|---|---|---|---|
| Initial Undigested | 33000 | 56000 | 11510 |
| % incorporation | 2% | 5% | 1% |
| Post DNase | 27000 | 49000 | 10000 |
| % of initial | 82% | 87% | 86% |
| Post RNase | 900 | 4500 | 100 |
| % of initial | 3% | 8% | 1% |

(d) RNA Transcripts as Probes

Southern blots of chlamydial DNA digested with eight restriction enzymes (see reference Example 2) were prehybridised with 10ml hydridisation buffer N (see Davis L.G. et al [1986] Basic Methods in Molecular Biology, Elsevier, London for hybridisation buffer N) at 60°C for one hour in sealed hybridisation bags. The two labelled RNA transcript samples (primer 9-ClaI and primer 8-ClaI) were heated to 94°C and added to 10mls fresh HBN and the blots were hybridised with these samples at 42°C overnight in sealed hybridisation bags. After washing twice in 2xSSC, 0.1% SDS and once in 0.1x SSC, 0.1% SDS at 55°C, the hybridised blots were sealed in hybridisation bags and autoradiographed with intensifying screens at -80°C for 48 hours.

The RNA probes transcribed from DEVIATS 9-ClaI (Figure 4) and 8-ClaI hybridised to Southern blots of restriction enzyme digests of C.Trachomatis $L_2$ DNA and gave good signal :noise ratios (Figures 9 and 10). The two probes hybridised with different ClaI and XbaI fragments as predicted from the published restriction map (Figure 4). Figure 9 shows an autoradiograph of a Southern blot of restriction enzyme digests of chlamydial DNA hybridised with a labelled RNA transcript from 9-ClaI DEVIATS. Approximate locations of size markers (bp) are shown to the side of the autoradiograph. The tracks have been labelled according to the restriction enzyme used for digestion of the C. trachomatis $L_2$ DNA : Sac(SacI), P(PstI), Sal(SalI), x(XbaI),H (HindIII), C-(ClaI), B(BamHI) and E(EcoRI).

Figure 10 shows an autoradiograph of a Southern blot of restriction enzyme digests of chlamydial DNA hybridised with a labelled RNA transcript from 8-ClaI DEVIATS. Approximate locations of size markers (bp) are shown to the side of the autoradiograph. The tracks have been labelled according to the restriction enzyme used for digestion of the C. trachomatis $L_2$ DNA : Sac(SacI), P(PstI), Sal(SalI), x(XbaI),H (HindIII), C(ClaI), B-(BamHI) and E(EcoRI).

Example 9

DEVIATS as an Alternative to Vectorettes for Chemical Genetics

PCR using MOMP specific primers (4, 5, 3, or 7) or a 23s rRNA -specific primer (10) with EcoR1 Vectorette and with EcoR1 DEVIATS Chlamydial libraries produced identical products as indicated in Figure 11 which shows amplification by PCR using specific primers with EcoRI Vectorette and DEVIATS chlamydial libraries and in which

Tracks 1 primer 4
2 primer 5
3 primer 3 + EcoRI DEVIATS Chlamydial library
4 primer 7
5 primer 10
6 primer 0x174 HaeIII size markers
7 primer 4
8 primer 5
9 primer 3 + EcoRI Vectorette Chlamydial library
10 primer 7
11 primer 10

Example 10

1µg of M13mp18 RF DNA was cut with XbaI and ligated to XbaI DEVIATS. Four aliquots, each having about 125ng of the ligated DNA, were annealed to one of four specific primers, numbers 11, 12, 13 and 14 at 500, 1000, 2000 or 4000 bp respectively from one end of the DEVIAT - ligated DNA. A single primer extension (4 minutes, 72°C) was done with Taq DNA polymerase in PCR buffer, the reaction stopped by phenol extraction, and the DNA separated from the reaction components by molecular sieve (Sephadex G-50). RNA transcripts were prepared with SP6 RNA polymerase and the level of incorporation of alpha $^{32}$P-ATP measured by TCA precipitation. The results show that all four primers were successful in producing a functional RNA Polymerase promoter (Figure 12). The level of incorporation was quite low, as was expected, due to the absence of PCR amplification and the TCA precipitated counts and % incorporation are shown in the Table below.

TABLE 2

| PRIMER | DISTANCE | TCA PRECIPITATED COUNTS | % INCORPORATION |
|---|---|---|---|
| 11 | 500bp | 85 | 0.03 |
| 12 | 1000bp | 286 | 0.09 |
| 13 | 2000bp | 703 | 0.23 |
| 14 | 4000bp | 486 | 0.16 |
| Positive Control | | 3700 | 1.30 |

It is therefore clear that it is not necessary to use PCR in the performance of the present invention to amplify a specific sequence as illustrated by the use of DEVIATS above.

Example 11

A vectorette library of Chlamydial DNA was made with restriction endonuclease Cla I as described in Reference Example 2 and using oligonucleotides 30 and 31. An aliquot of this library was amplified by the polymerase chain reaction (PCR) using oligonucleotides 32 and 33 as amplification primers under the conditions described in Reference Example 3. 10µl of the PCR reaction product were incubated with 10 units of Bam HI restriction endonuclease. To this were added 5µl of a mixture containing 1µg of pAT153 plasmid DNA, previously digested with Bam HI and Pst I in restriction buffer (according to enzyme manufacturers instructions). The solution containing pAT153 and PCR product were heated at 70°C for 10 minutes. Adenosine triphosphate (ATP) and dithiothreitol (DTT) were added to a final concentration of 1mM and 10 units of T4 DNA ligase were then added.

This was incubated for 1 hour. The sample was then analysed by electrophoresis on a 1% agarose gel.

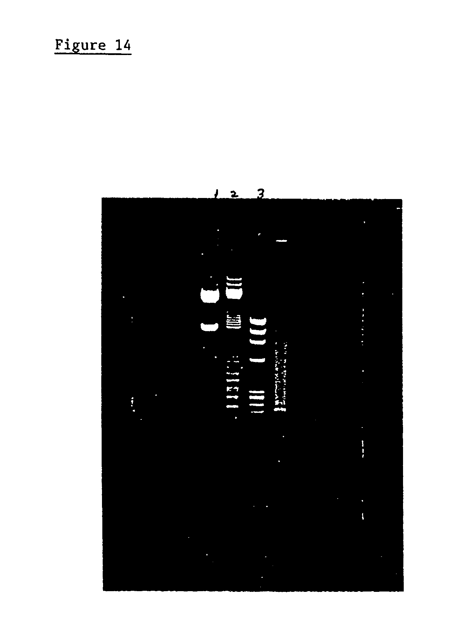

The results are shown in Figure 14. Track 1 illustrates pAT153 digested with Bam HI and Pst I. Track 2 shows a ligation mixture containing pAT153, Pst I and Bam HI digest and PCR product Bam HI digest. Track 3 shows a Phix 174 Hae III digest (markers). The results show that the restriction site in the Vectorette 2 sequence (created using oligonucleotides 30 and 31) can be digested and religated.

**SEQUENCE LISTING**

**(1) GENERAL INFORMATION**

(i) **APPLICANT :** Imperial Chemical Industries PLC
(ii) **TITLE OF INVENTION :** AMPLIFICATION METHODS
(iii) **NUMBER OF SEQUENCES :** 34
(iv) **CORRESPONDENCE ADDRESS :**
(A) **ADDRESSEE :** Legal Department : Patents
(B) **STREET :** Bessemer Road
(C) **CITY :** Welwyn Garden city
(D) **STATE :** Hertfordshire
(E) **COUNTRY :** United Kingdom
(F) **ZIP :** GB-AL7 1HD
(v) **COMPUTER READABLE FORM :**
(A) **MEDIUM TYPE :** Diskette, 5.25 inch, 1.2 Mb storage
(B) **COMPUTER :** Tandon
(C) **OPERATING SYSTEM :** PC-DOS 3.20
(D) **SOFTWARE :** ASCII from WPS-PLUS
(vi) **CURRENT APPLICATION DATA :**
(A) **APPLICATION NUMBER**
(B) **FILING DATE :**
(vii) **PRIOR APPLICATION DATA :**
(A) **APPLICATION NO.** 9001764.1
(B) **FILING DATE :** 25-Jan-1990
(A) **APPLICATION NO.** 9025283.4
(B) **FILING DATE :** 21-Nov-1990

SEQ ID No 1
SEQUENCE LENGTH : 30
SEQUENCE TYPE : Nucleotide
STRANDEDNESS : Single
TOPOLOGY : Linear

CGAATCGTAA CCGTTCGTAC GAGAATCGCT 30

SEQ ID No 2
SEQUENCE LENGTH : 20
SEQUENCE TYPE : Nucleotide
STRANDEDNESS : Single
TOPOLOGY : Linear

AGAATCGCTG TCCTCTCCTT 20

SEQ ID No 2a
SEQUENCE LENGTH : 15
SEQUENCE TYPE : Nucleotide
STRANDEDNESS : Single
TOPOLOGY : Linear

CGCTGTCCTC TCCTT 15

SEQ ID No 3
SEQUENCE LENGTH : 25
SEQUENCE TYPE : Nucleotide
STRANDEDNESS : Single
TOPOLOGY : Linear

CTGCTCACGT AAATGCACAA TTCCG 25

SEQ ID No 4

SEQUENCE LENGTH : 25
SEQUENCE TYPE : Nucleotide
STRANDEDNESS : Single
TOPOLOGY : Linear

TGAAATCGGT ATTAGTGTTT GCCGC 25

SEQ ID No 5
SEQUENCE LENGTH : 25
SEQUENCE TYPE : Nucleotide
STRANDEDNESS : Single
TOPOLOGY : Linear

GTGCATTTAC GTGAGCAGCT CTCTC 25

SEQ ID No 6
SEQUENCE LENGTH : 24
SEQUENCE TYPE : Nucleotide
STRANDEDNESS : Single
TOPOLOGY : Linear

CCTGAAGGGC GCACAGTAGC TGAT 24

SEQ ID No 7
SEQUENCE LENGTH : 24
SEQUENCE TYPE : Nucleotide
STRANDEDNESS : Single
TOPOLOGY : Linear

CAAGGCATAA CGTGTTGATT GGTG 24

SEQ ID No 8
SEQUENCE LENGTH : 25
SEQUENCE TYPE : Nucleotide
STRANDEDNESS : Single
TOPOLOGY : Linear

GGGTCTGATC CACCAGACTA TTTCT 25

SEQ ID No 9
SEQUENCE LENGTH : 25
SEQUENCE TYPE : Nucleotide
STRANDEDNESS : Single
TOPOLOGY : Linear

CTTCCGATAC ATTGACTGTT CCAGT 25

SEQ ID No 10
SEQUENCE LENGTH : 21
SEQUENCE TYPE : Nucleotide
STRANDEDNESS : Single
TOPOLOGY : Linear

CGTTCTCATC GCTCTACGGA C 21

SEQ ID No 11
SEQUENCE LENGTH : 25
SEQUENCE TYPE : Nucleotide
STRANDEDNESS : Single
TOPOLOGY : Linear

TTGACGTTGG AGTCCAGGTT CTTTA 25

SEQ ID No 12
SEQUENCE LENGTH : 25
SEQUENCE TYPE : Nucleotide
STRANDEDNESS : Single
TOPOLOGY : Linear

GAGTTCTTCT ACTCAGGCAA GTGAT 25

SEQ ID No 13
SEQUENCE LENGTH : 25
SEOUENCE TYPE : Nucleotide
STRANDEDNESS : Single
TOPOLOGY : Linear

ATTCGCCTCT GCGCGATTTT GTAAC 25

SEQ ID No 14
SEQUENCE LENGTH : 25
SEQUENCE TYPE : Nucleotide
STRANDEDNESS : Single
TOPOLOGY : Linear

TGTGAATATC AAGGCCAATC GTCTG 25

SEQ ID No 15
SEQUENCE LENGTH : 43
SEQUENCE TYPE : Nucleotide
STRANDEDNESS : Single
TOPOLOGY : Linear

AATTGCAGGA GACCCATTT AGGTGACACT ATAGAATACG GCG 43

SEQ ID No 16
SEQUENCE LENGTH : 43
SEQUENCE TYPE : Nucleotide
STRANDEDNESS : Single
TOPOLOGY : Linear

CTAGCGCCGT ATTCTAATAC GACTCACTAT AGGGTTCTCC TGC 43

SEQ ID No 17
SEQUENCE LENGTH : 43
SEQUENCE TYPE : Nucleotide
STRANDEDNESS : Single
TOPOLOGY : Linear

GATCGCAGGA GAACCCATTT AGGTGACACT ATAGAATACG GCG 43

SEQ ID No 18
SEQUENCE LENGTH : 41
SEQUENCE TYPE : Nucleotide
STRANDEDNESS : Single
TOPOLOGY : Linear

CGCGCCGTAT TCTAATACGA CTCACTATAG GGTTCTCCTG C 41

SEQ ID No 19
SEQUENCE LENGTH : 17
SEQUENCE TYPE : Nucleotide
STRANDEDNESS : Single
TOPOLOGY : Linear

ACCCATTTAG GTGACAC 17

SEQ ID No 20
SEQUENCE LENGTH : 17
SEQUENCE TYPE : Nucleotide
STRANDEDNESS : Single
TOPOLOGY : Linear

TTCTAATACG ACTCACT 17

SEQ ID No 21
SEQUENCE LENGTH : 58
SEQUENCE TYPE : Nucleotide
STRANDEDNESS : Single
TOPOLOGY : Linear

GATCGAAGGA GAGGACGCTG TCTGTCGAAG GTAAAGGAAC GGAGGAGAGA AGGGAGAG 58

SEQ ID No 22
SEQUENCE LENGTH : 53
SEQUENCE TYPE : Nucleotide
STRANDEDNESS : Single
TOPOLOGY : Linear

CTCTCCCTTC TCGAATCGTA ACCGTTCGTA CGAGAATCGC TGTCCTCTCC TTC 53

SEQ ID No 23
SEQUENCE LENGTH : 55
SEQUENCE TYPE : Nucleotide

STRANDEDNESS : Single
TOPOLOGY : Linear
CGGAAGGAGA GGACGCTGTC TGTCGAAGGT AAGGAACGGA GGAGAGAAGG GAGAG 55
SEQ ID No 24
SEQUENCE LENGTH : 57
SEQUENCE TYPE : Nucleotide
STRANDEDNESS : Single
TOPOLOGY : Linear
AGCTGAAGGA GAGGACGCTG TCTGTCGAAG GTAAGGAACG GAGGAGAGAA GGGAGAG 57
SEQ ID No 25
SEQUENCE LENGTH : 57
SEQUENCE TYPE : Nucleotide
STRANDEDNESS : Single
TOPOLOGY : Linear
CTAGGAAGGA GAGGACGCTG TCTGTCGAAG GTAAGGAACG GAGGAGAGAA GGGAGAG 57
SEQ ID No 26
SEQUENCE LENGTH : 57
SEQUENCE TYPE : Nucleotide
STRANDEDNESS : Single
TOPOLOGY : Linear
TCGAGAAGGA GAGGACGCTG TCTGTCGAAG GTAAGGAACG GAGGAGAGAA GGGAGAG 57
SEQ ID No 27
SEQUENCE LENGTH : 53
SEQUENCE TYPE : Nucleotide
STANDEDNESS : Single
TOPOLOGY : Linear
AAAGGAGAGG ACGCTGTCTG TCGAAGGTAA GGAACGGAGG AGAGAAGGGA GAG 53
SEQ ID No 28
SEQUENCE LENGTH : 57
SEQUENCE TYPE : Nucleotide
STRANDEDNESS : Single
TOPOLOGY : Linear
CTCTCCCTTC TCGAATCGTA ACCGTTCGTA CGAGAATCGC TGTCCTCTCC TTTTGCA 57
SEQ ID No 29
SEQUENCE LENGTH : 57
SEQUENCE TYPE : Nucleotide
STRANDEDNESS : Single
TOPOLOGY : Linear
CTCTCCCTTC TCGAATCGTA ACCGTTCGTA CGAGAATCGC TGTCCTCTCC TTTAGCT 57
SEQ ID No 30
SEQUENCE LENGTH : 54
SEQUENCE TYPE : Nucleotide
STRANDEDNESS : Single
TOPOLOGY : Linear
CGGCAGGAGA ACCCCAGAGT TCGGGATGAT GCTTGCTAGA TGTAGAATAC GGCG 54
SEQ ID No 31
SEQUENCE LENGTH : 53
SEQUENCE TYPE : Nucleotide
STRANDEDNESS : Single
TOPOLOGY : Linear
CGCCGTATTC TAGTACTTCG AACCTTAAGC CCTAGGTGCA GGGTTCTTCC TGC 53
SEQ ID No 32
SEQUENCE LENGTH : 25
SEQUENCE TYPE : Nucleotide
STRANDEDNESS : Single
TOPOLOGY : Linear
CTTCGAACCT TAAGCCCTAG GTGCA 25
SEQ ID No 33

SEQUENCE LENGTH : 25
SEQUENCE TYPE : Nucleotide
STRANDEDNESS : Single
TOPOLOGY : Linear

GAATTGACGG TGAATGTACA TAACG 25

## Claims

1. A method for the amplification of at least a part of a target nucleic acid fragment/vectorette unit, said part comprising i) unknown sequence, ii) a first priming region of known nucleotide sequence for hybridisation with a first primer and iii) a vectorette portion at least one strand of which has a nucleic acid sequence which in either its double stranded complementary form or its single stranded form is capable of being bound by protein, which method comprises treating the target nucleic acid fragment/vectorette unit, together or sequentially, with appropriate nucleoside triphosphates and an agent for polymerisation of the nucleoside triphosphates under hybridising conditions, such that an extension product of a first priming region is synthesised complementary to a single stranded target nucleic acid/vectorette unit having a first priming region to which is hybridised a first primer selected so as to be substantially complementary to the first priming region, whereas no such extension product is synthesised complementary to single stranded target nucleic acid fragment/vectorette units having no such first priming region.

2. A method as claimed in claim 1 wherein at least a part of the vectorette unit(s) is amplified by a) primer extension of a first primer, said first primer being hybridised to the first priming region of a nucleic acid sequence which sequence comprises i) unknown sequence and ii) a promoter sequence ; followed by b) repeated generation of nucleic acid sequence under the control of the said promoter sequence.

3. A method as claimed in claim 1 or claim 2 wherein the synthesis of vectorette primer amplification products is dependent upon the initial synthesis of an extension product of the initiating primer.

4. A method as claimed in any one of claims 1-3 wherein at least one vectorette portion of the target nucleic acid fragment/vectorette unit comprises a double stranded portion having first and second strands, the first strand having a terminal polymerisation blocking moiety and the second strand carrying a single stranded portion which comprises nucleic acid sequence either in its double stranded complementary form or in its single stranded form is capable of being bound by protein, the second strand being ligated to that strand of the target nucleic acid fragment containing a first priming region for hybridisation with a first primer, the terminal polymerisation blocking moiety being effective to prevent extension of the first strand to form a complement to the said single stranded portion of the second strand in the presence of appropriate nucleoside triphosphates and an agent for the polymerisation of the nucleoside triphosphates under hybridising conditions.

5. A method as claimed in claim 4 wherein the vectorette portion of the target nucleic acid fragment/vectorette unit comprises a double stranded portion having first and second strands, the second strand of the vectorette portion being ligated to that strand of the target nucleic acid fragment which contains the initiating priming region, and the nucleotide sequence of the first strand, second strand and vectorette primer being selected such that the vectorette primer is capable of hybridising to the complement of the second strand but not to the first strand under the same hybridisation conditions.

6. A method as claimed in any one of the preceding claims wherein a plurality of different vectorette libraries is prepared for use with the same single initiating primer, each vectorette library comprising target nucleic acid fragment/vectorette units obtained by ligation of nucleic acid fragments prepared by cleaving target nucleic acid at different cleavage sites ; treating each vectorette library either separately or together with appropriate nucleoside triphosphates and an agent for polymerisation of nucleoside triphosphates under hybridising conditions whereby to obtain a plurality of initiating primer extension products, based on the use of the same single initiating primer.

7. A method as claimed in any one of the previous claims wherein the nucleic acid sequence is only capable of being bound by protein when in its double stranded complementary form.

8. A method as claimed in claim 7 wherein the nucleic acid sequence is capable of being bound by a restriction enzyme.

9. A method as claimed in any one of claims 1-7 wherein the nucleic acid sequence is capable of binding a DNA or RNA polymerase.

10. A method as claimed in claim 9 wherein the nucleic acid sequence is capable of binding SP6 or T7 RNA polymerase.

11. A method for the production of a target nucleic acid fragment/vectorette unit having a vectorette portion at each terminus of the said unit which method comprises cleaving a target nucleic acid fragment/vectorette unit in a region of unknown sequence in the target nucleic acid fragment portion of the said vectorette unit and ligating a vectorette to the cleaved target nucleic acid portion obtained to form a vectorette unit having a vectorette portion at each terminus of the said unit.

12. A method as claimed in claim 2 wherein the vectorette is as stated in any one of claims 1-10.

13. A vectorette comprising double stranded nucleic acid having a region of non-complementarity, this region having in a first strand a nucleic acid sequence which in either its double stranded complementary form or in its single stranded form is capable of being bound by one protein and this region having in a second strand a nucleic acid sequence which in either its double stranded complementary form or in its single stranded form is capable of being bound by a protein different from that which is capable of binding the nucleic acid sequence in the first strand.

14. A vectorette as claimed in claim 13 and comprising nucleic acid sequence(s) as stated in any one of claims 7-10.

15. A kit for the amplification of a nucleic acid fragment of unknown sequence by primer extension, which kit comprises a vectorette adapted for ligation to a target nucleic acid fragment or for ligation to a target nucleic acid fragment/vectorette unit, said fragment or vectorette unit having been obtained by use of a means for cleaving a target nucleic acid whereby to form in use a target nucleic acid fragment/vectorette unit having either a single terminal vectorette or a vectorette at each terminus of the target nucleic acid fragment said vectorette being such that at least one strand thereof has a nucleic acid sequence which in either its double stranded complementary form or its single stranded form is capable of being bound by protein, and written or printed instructions for use of the kit, and further includes one or more of the following optional items :- (a) the appropriate protein which is capable of binding the nucleic acid sequence, (b) means for cleaving a target nucleic acid at a specific site to obtain a target nucleic acid fragment or to obtain a target nucleic acid fragment/vectorette unit, (c) each of four different nucleoside triphosphates, (d) an agent for polymerisation of the nucleoside triphosphates in (b), (e) appropriate first and/or vectorette primers, and (f) appropriate buffers.

16. A kit as claimed in claim 15 and comprising one or more of the following :- first primer(s), nested vectorette primer(s), nested first primer(s), sequencing primers, buffers for performing the method of the present invention, and buffers for varying the magnesium, potassium and nucleoside triphosphate concentrations.

17. A vectorette library kit for the amplification of a nucleic acid fragment of unknown sequence by primer extension, which kit comprises :-
1) at least one vectorette library each library comprising a set of target nucleic acid fragment/vectorette units obtained from nucleotide sequence of an individual member of a species of animal, plant or microorganism, each unit comprising target nucleic acid having either one or two terminal vectorette portions, at least one of said portions having at least one strand of nucleic acid sequence which in either its double stranded complementary form or its single stranded form is capable of being bound by protein ; and
2) a first primer or primers for hybridisation to the first priming region of the target nucleic acid fragment/vectorette units.

18. A kit or vectorette library kit as claimed in any one of claims 15-16 or claim 17 wherein the vectorette(s) comprise nucleic acid sequence(s) as stated in any one of claims 7-10.

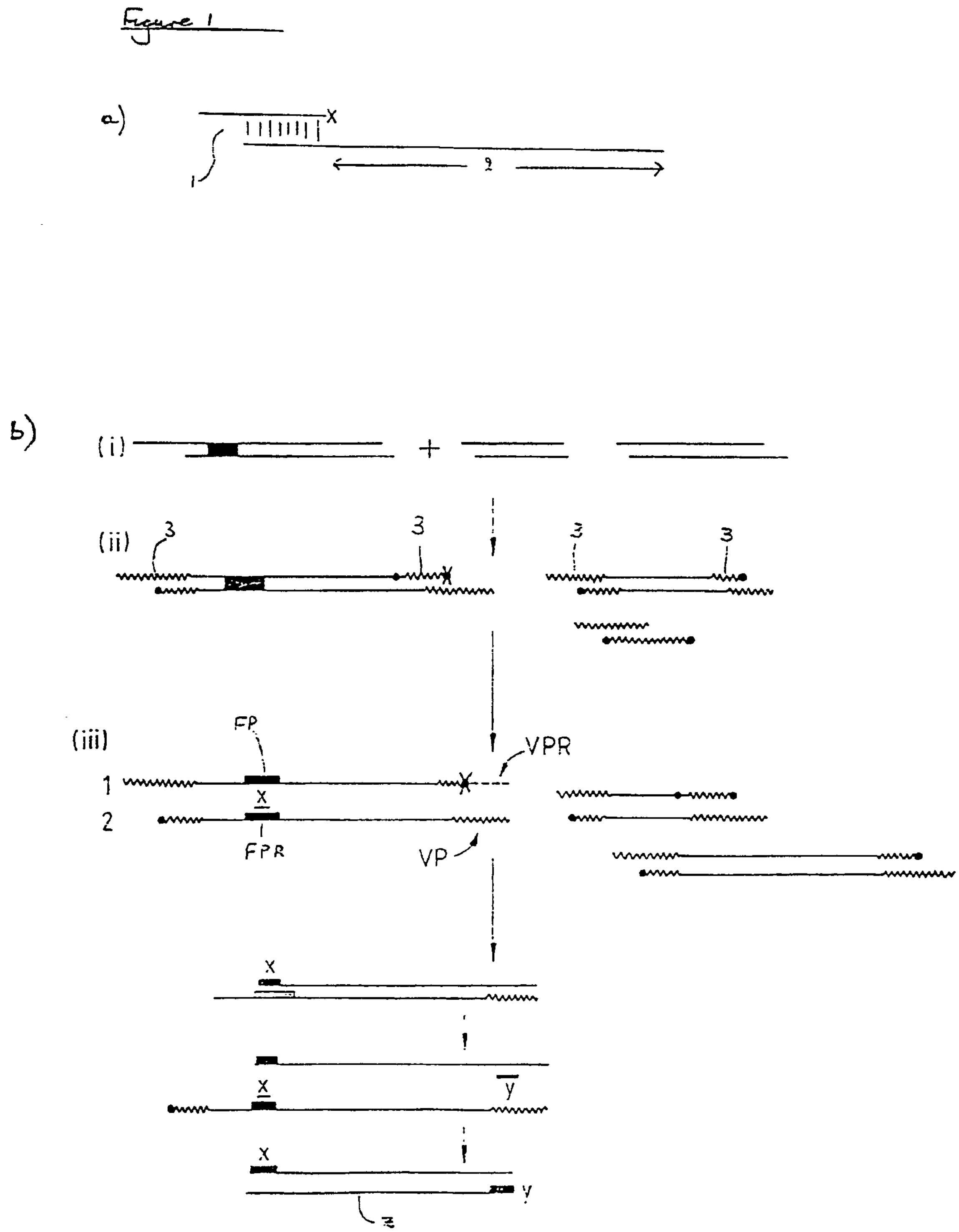
Figure 1
a)
X
1
2
b)
(i)
(ii)
3
3
3
3
(iii)
FP
VPR
1
X
2
FPR
VP
X
X
y
X
y
z

Figure 2

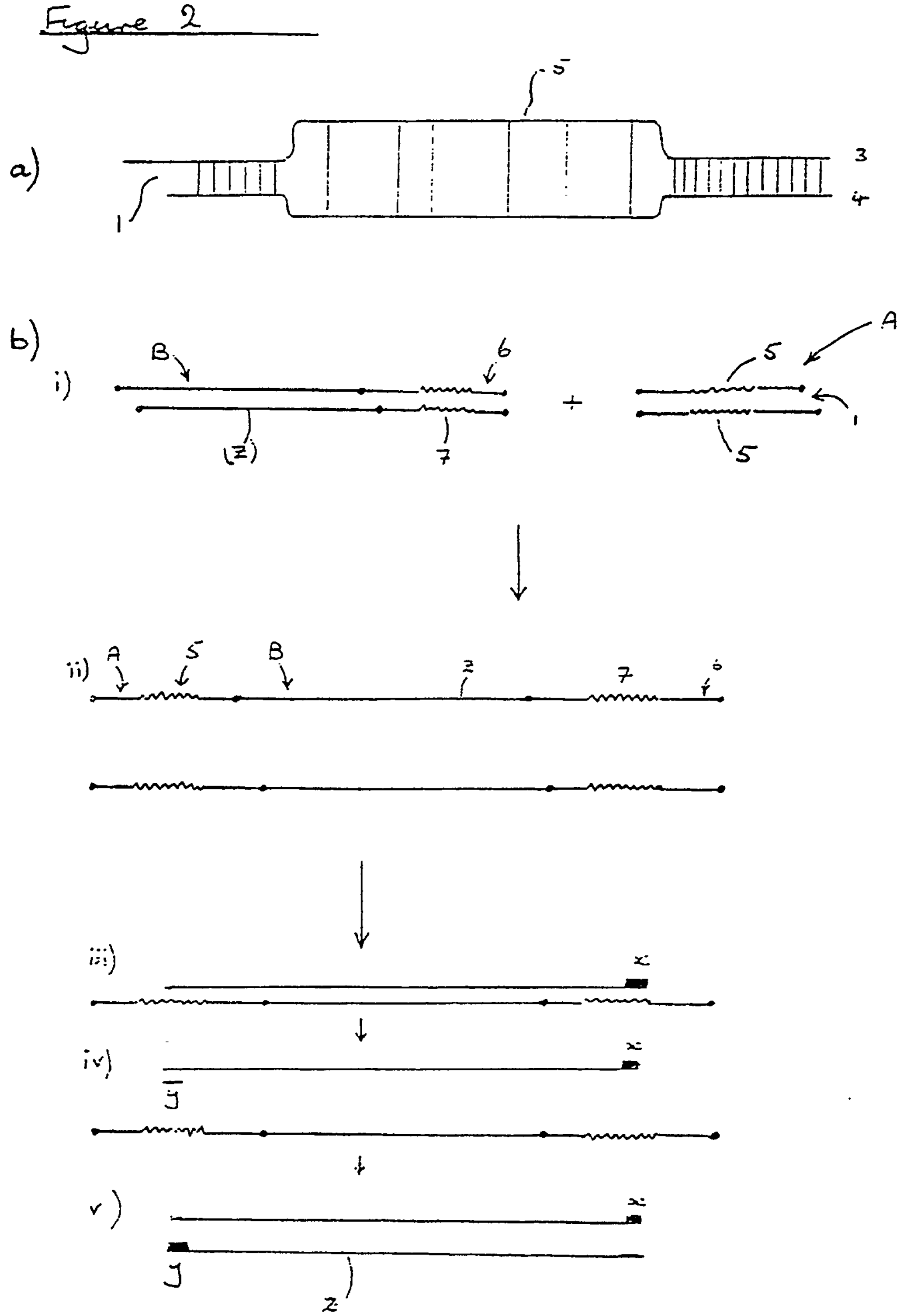

Figure 3

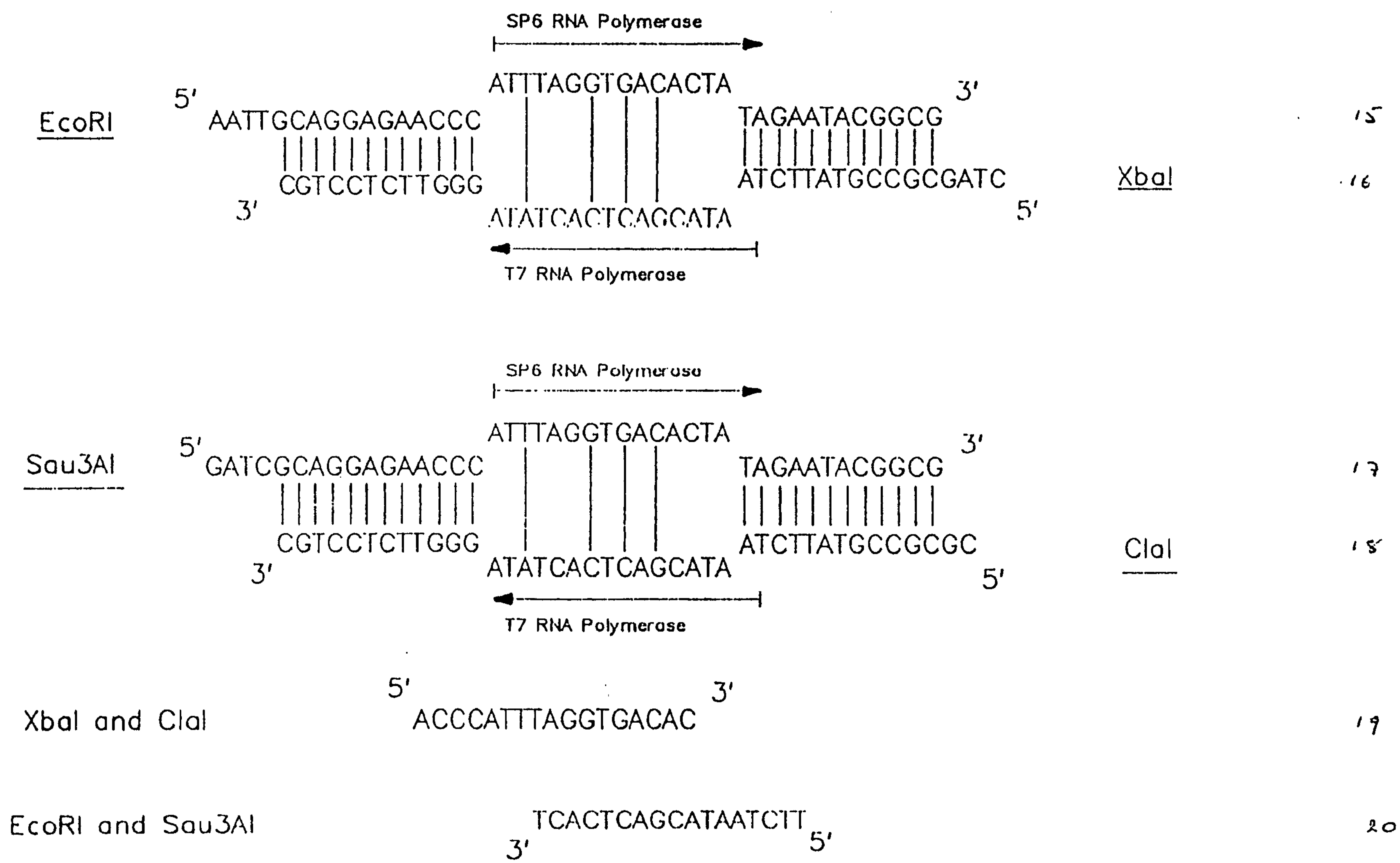
SP6 RNA Polymerase
EcoRI
5' AATTGCAGGAGAACCC
3' CGTCCTCTTGGG
ATTTAGGTGACACTA
ATATCACTCAGCATA
TAGAATACGGCG 3'
ATCTTATGCCGCGATC 5'
T7 RNA Polymerase
XbaI
15
16
SP6 RNA Polymerase
Sau3AI
5' GATCGCAGGAGAACCC
3' CGTCCTCTTGGG
ATTTAGGTGACACTA
ATATCACTCAGCATA
TAGAATACGGCG 3'
ATCTTATGCCGCGC 5'
T7 RNA Polymerase
ClaI
17
18
XbaI and ClaI
5' ACCCATTTAGGTGACAC 3'
19
EcoRI and Sau3AI
3' TCACTCAGCATAATCTT 5'
20

Figure 4

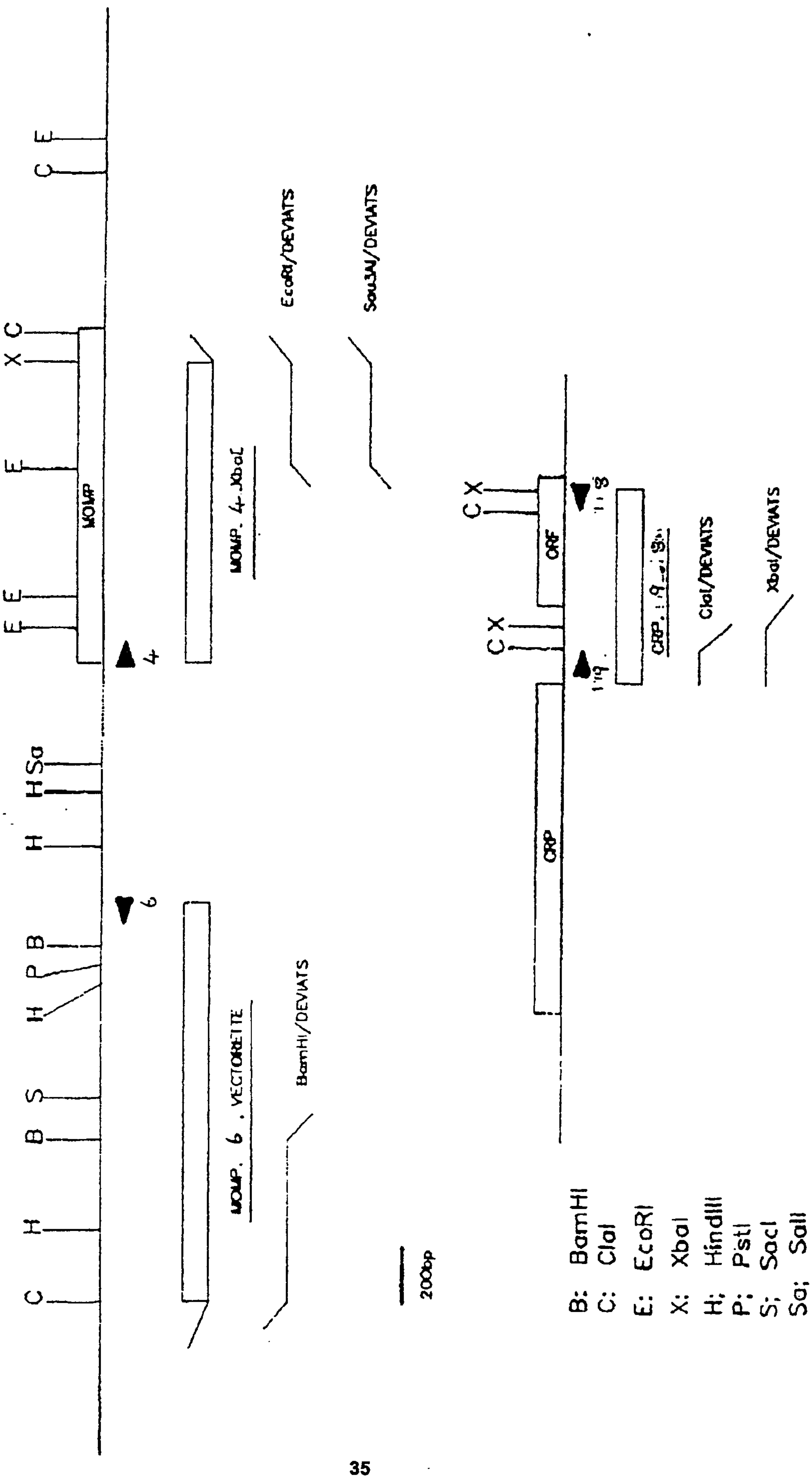
WOMP
WOMP. 4. XbaI
EcoRI/DEVIATS
Sau3AI/DEVIATS
WOMP. 6. VECTORETTE
BamHI/DEVIATS
CRP
ORF
ClaI/DEVIATS
XbaI/DEVIATS
200bp
B: BamHI
C: ClaI
E: EcoRI
X: XbaI
H: HindIII
P: PstI
S: SacI
Sa: SalI

Figure 5

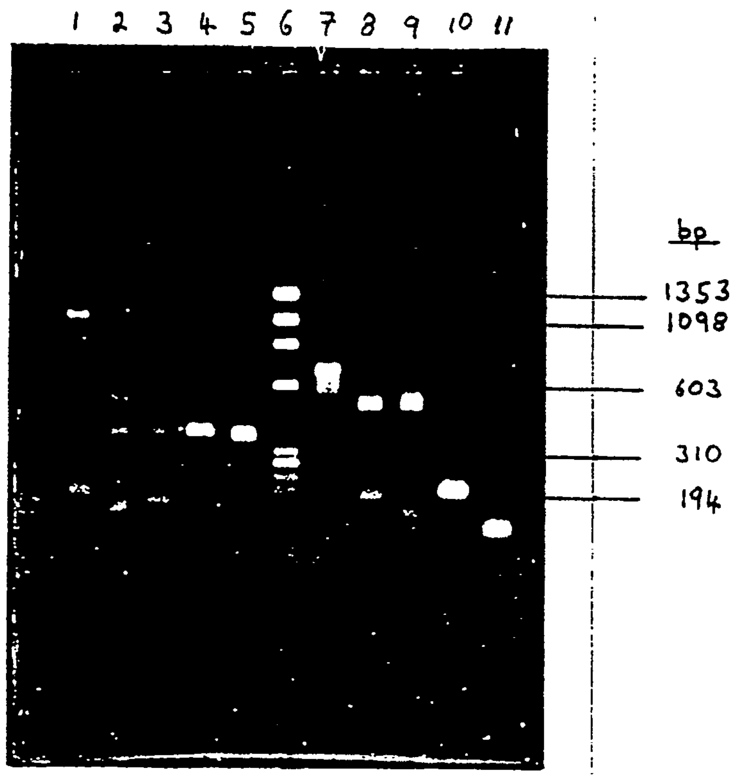

Figure 6

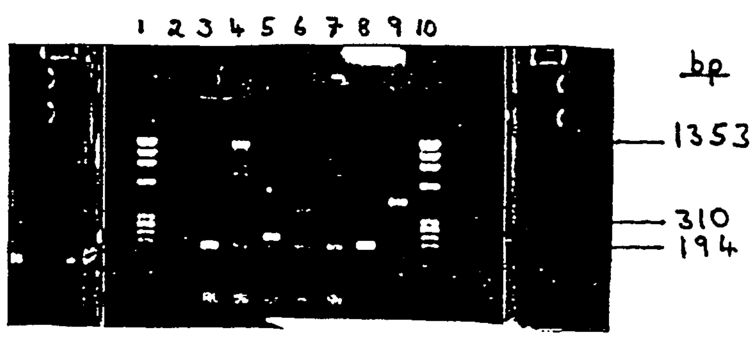

Figure 7

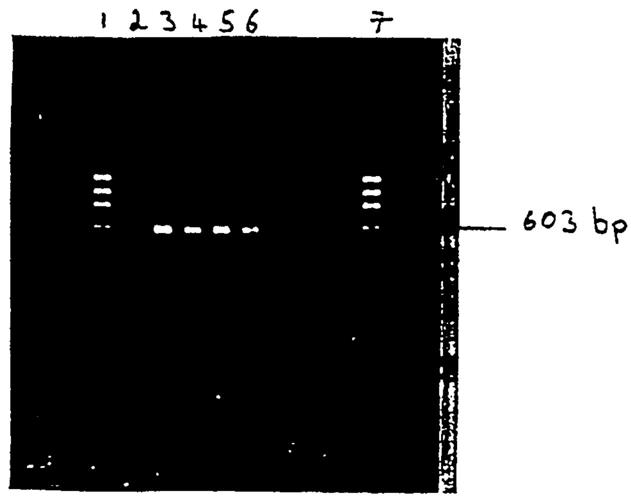

Figure 8

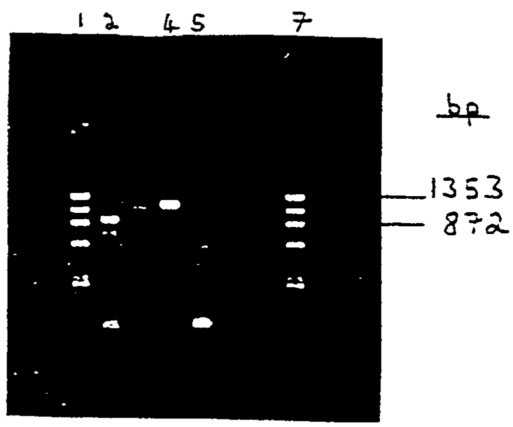

Figure 9

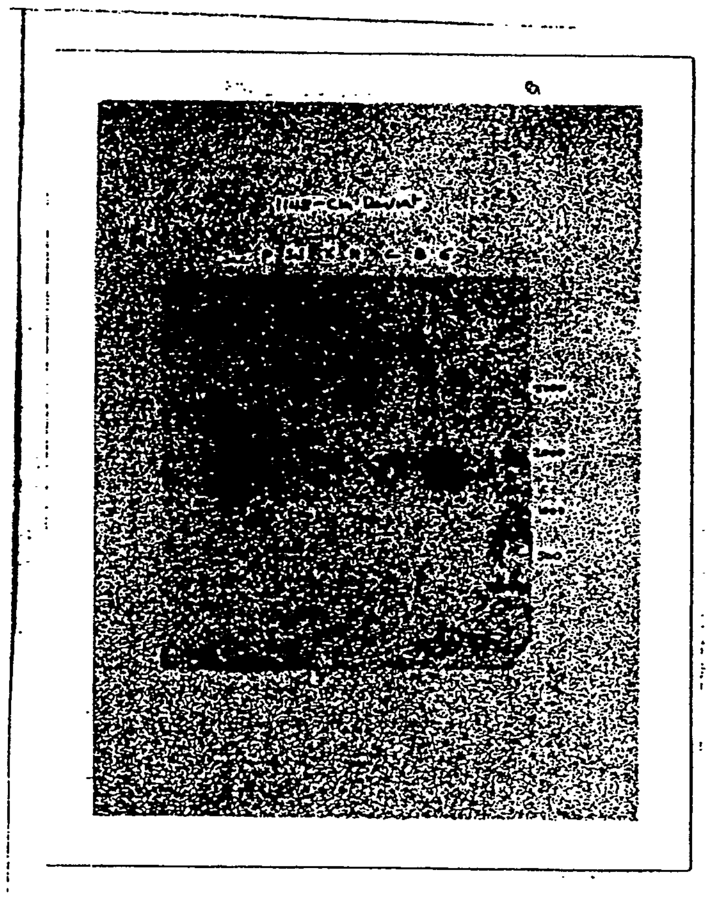

Figure 10

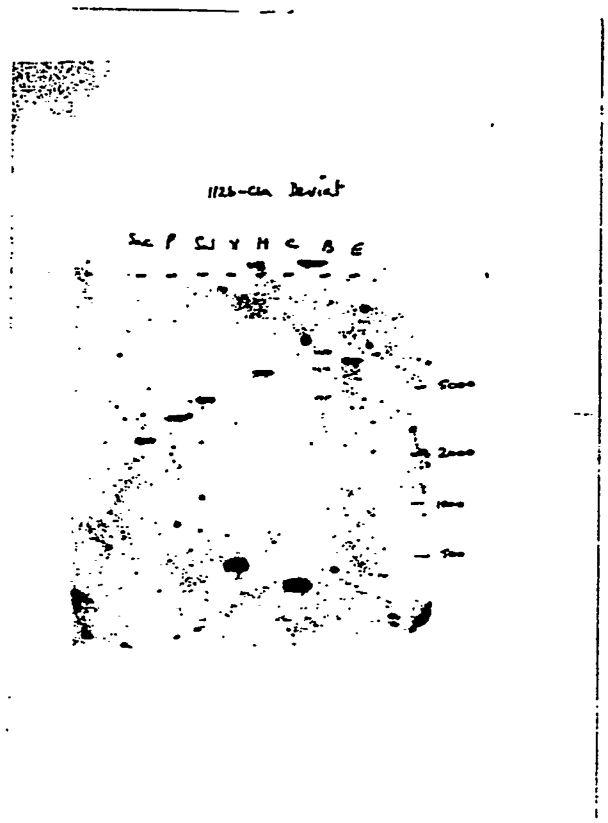

Figure 11

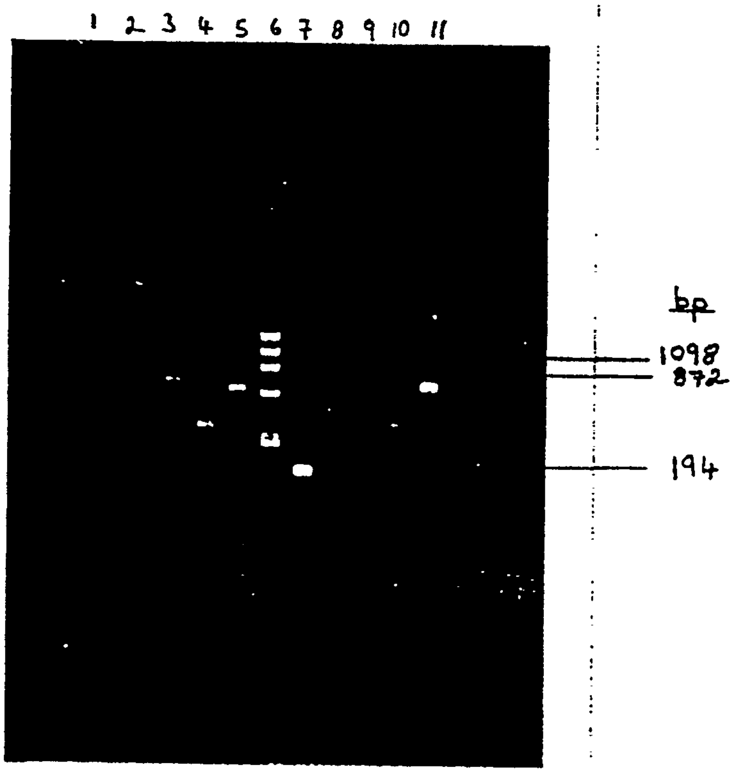

Figure 12

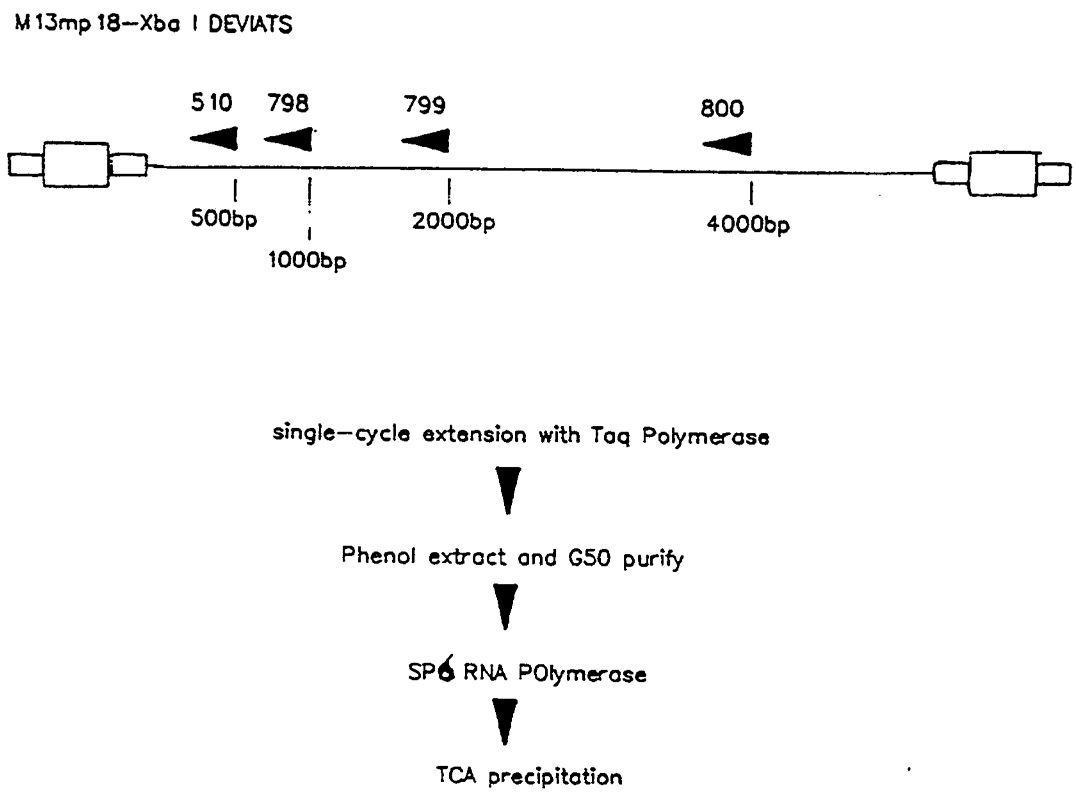
M 13mp 18–Xba I DEVIATS
510
798
799
800
500bp
1000bp
2000bp
4000bp
single–cycle extension with Taq Polymerase
Phenol extract and G50 purify
SP6 RNA POlymerase
TCA precipitation

FIGURE 13

1 AAATGCATTCTCCATCAGTTTAGCAAATGATTTCCGCAAAGCAATGAAAT

51 GCCCTCGGATCAATCTACAAAATCCAAGACAGACATATGGCCAATTTCTG

101 CTAATAAAGAGATAGAGACTTTTTCTGaccttgaCTACGCAATAgATCCt

151 agGAAATAGacagTTTTATCGtcaGTTGGCAGATacGATCTGCAAAAATA

201 GAGCCAAGACGCTCGCGaacaAaGCTTGTACCTCTTCTTCTCTTtCAGCA

251 AGGATGACTTtaAACTGTTtCTgTCGTTtAAGAGATGTGACCTcTCcGAT

301 AATCTCATGCCCATCCTCTTTAGGGAATAGACATACAAAACGAGACCACA

351 CTCTTTTCTCACATCAACATTCAACCTGCTCGATCGTTCCTTGCAT

Figure 14

1 2 3